(19) Europäisches Patentamt
European Patent Office
Office européen des brevets

(11) EP 3 977 935 A1

(12) EUROPEAN PATENT APPLICATION
published in accordance with Art. 153(4) EPC

(43) Date of publication:
**06.04.2022 Bulletin 2022/14**

(21) Application number: **20813616.8**

(22) Date of filing: **27.05.2020**

(51) International Patent Classification (IPC):
***A61B 6/00** (2006.01)* ***A61B 6/03** (2006.01)*
***A61B 6/14** (2006.01)* ***G01N 23/044** (2018.01)*
***G01N 23/087** (2018.01)*

(52) Cooperative Patent Classification (CPC):
**A61B 6/00; A61B 6/03; A61B 6/14; G01N 23/044; G01N 23/087**

(86) International application number:
**PCT/JP2020/020870**

(87) International publication number:
**WO 2020/241669 (03.12.2020 Gazette 2020/49)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **27.05.2019 JP 2019098358**

(71) Applicant: **Diatrend Corporation**
**Osaka-shi, Osaka 530-0011 (JP)**

(72) Inventors:
- **YAMAKAWA, Tsutomu**
**Nishinomiya-shi, Hyogo 662-0875 (JP)**
- **MIYASHITA, Sugaya**
**Osaka-shi, Osaka 530-0011 (JP)**
- **OOSUGI, Jun**
**Osaka-shi, Osaka 530-0011 (JP)**
- **SAKAMOTO, Kyouhei**
**Osaka-shi, Osaka 530-0011 (JP)**
- **HAYAKAWA, Ryutaro**
**Nagareyama-shi, Chiba 270-0114 (JP)**

(74) Representative: **Kuhnen & Wacker**
**Patent- und Rechtsanwaltsbüro PartG mbB**
**Prinz-Ludwig-Straße 40A**
**85354 Freising (DE)**

(54) **DATA PROCESSING DEVICE AND DATA PROCESSING METHOD FOR PROCESSING X-RAY DETECTION DATA AND X-RAY INSPECTION APPARATUS PROVIDED WITH DEVICE AND METHOD**

(57) A data processing device is provided which irradiates an object with continuous X-rays and processes the data obtained from the X-ray transmission data detected by a photon counting X-ray detection device. An n-dimensional vector corresponding to each of the n energy regions set for the continuous X-rays, corresponding to the line attenuation value of the X-rays as they penetrate the object, is calculated for each detector pixel based on the aforementioned data. For each of the plurality of search regions virtually set up based on one or more detector pixels in the plurality of detector pixels, the n-dimensional vectors of the detector pixels belonging to each of the said plurality of search pixels are mutually vector added in the n-dimensional space. As a result, the n-dimensional representative vector representing each of the plurality of search regions is calculated for each of the search regions. Based on the representative vectors of each search region and the unit region of the desired size virtually set in the material space with the coordinate information of the degree of attenuation of the X-ray when the X-ray penetrates the object, the information indicating the amount, type and properties of the material of the object is obtained.

EP 3 977 935 A1

FIG.1

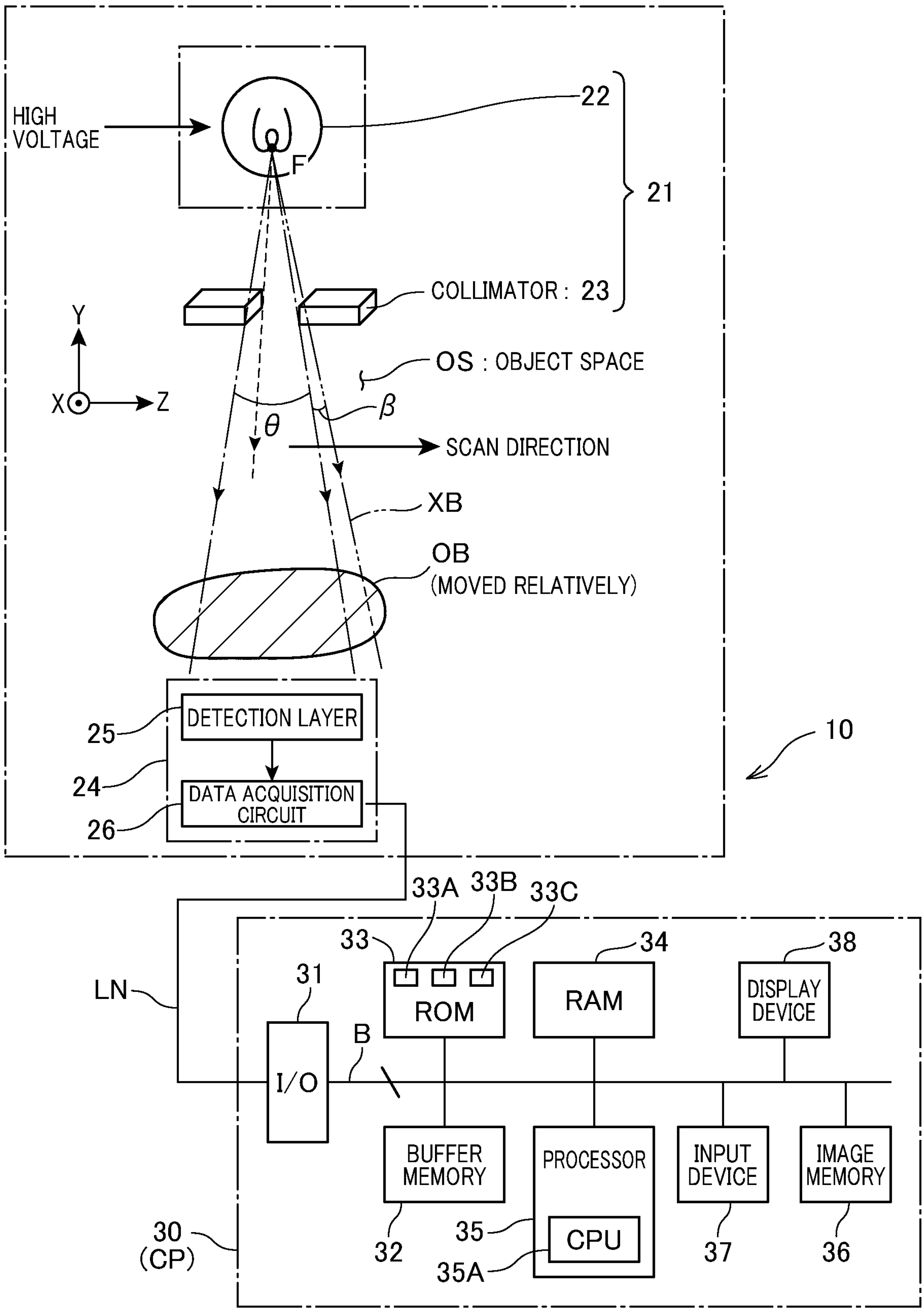

HIGH VOLTAGE
F
22
21
COLLIMATOR : 23
Y
X
Z
OS : OBJECT SPACE
θ
β
SCAN DIRECTION
XB
OB
(MOVED RELATIVELY)
25
DETECTION LAYER
24
DATA ACQUISITION CIRCUIT
26
10
33A 33B 33C
33
ROM
34
RAM
38
DISPLAY DEVICE
LN
31
I/O
B
BUFFER MEMORY
PROCESSOR
CPU
INPUT DEVICE
IMAGE MEMORY
30 (CP)
32
35
35A
37
36

## Description

[Technical field]

The present invention relates to a data processing device and a data processing method which are both for processing X-ray detection data obtained by irradiating X-rays to a target being inspected and by arcaturing the X-rays transmitted through the object, and an X-ray system equipped with such a device or by which such a method is adopted.

[Related art]

Conventionally, an X-ray inspection such as a medical diagnosis and a non-destructive inspection have been performed using continuous X-rays. In this X-ray inspection, an object being inspected is irradiated with X-rays, and X-rays transmitted through the object are detected by a planar detector, which is referred to as spot imaging. On the other hand, in recent years, a new inspection method has been proposed in this field of this X-ray inspection. In this inspection method, the object is scanned with an X-ray beam, and X-ray detection data is acquired. Based on this acquired X-ray inspection data, an image showing the inside of the object is reconstructed, and/or the state of the inside of the object is evaluated from a viewpoint of an elemental (material) level.

Conventional devices for diagnosing and/or inspecting the inside of a patient's body or an object using continuous X-rays are diverse, including radiography systems, X-ray CT scanners, and in-line non-destructive X-ray inspection systems. However, these devices collect X-rays, i.e., X-ray photons (simply, photons), by integrating the X-rays at regular intervals. Therefore, the X-ray dose attenuated based on the X-ray linear attenuation coefficients of one or more elements in the patient's body or the object is collected as an integrated value, and such integrated values are converted into an image or other information.

The photons of continuous X-rays emitted from an X-ray tube have various energies in the range up to the X-ray energy corresponding to the tube voltage, when being viewed on a particle-by-particle basis. If the energy amounts of the X-ray particles are different from each other, the attenuation states of the X-rays received from the elements (materials) in the object will be different from each other. Based on this respect, in recent years, X-ray inspection using a photon counting detector provided with energy discrimination function has been attracting attention, as shown in patent document 1.

In this X-ray inspection, the number of X-ray photons (simply, photons) transmitted through an object is measured (counted) in each of a plurality of energy ranges (i.e., energy bin), and the measurement results are used. In terms of using the plurality of energy ranges, a diagnostic method called a DEXA (or subtraction) method is also known, as shown in non-patent document 1.

In this situation, the X-ray inspection method described in patent document 2 has been proposed. The X-ray inspection method described in this document 2 is adapted to a configuration that uses a photon counting detector with the energy discrimination function described above.

This will be explained in detail. According to the configuration of this patent document 2, X-rays irradiated from an X-ray tube (21) are transmitted through an object and are detected by a photon counting detection unit (26). These detected X-rays are acquired as photon counts, for example, for each of the three energy ranges and for each pixel. An image of the object (OB) is calculated based on the measured values, and a region of interest is defined on the image. In addition, the background pixel information of a substance (consisting of one or more elements) in the region of interest is removed from the image. Then, based on the counted values of each X-ray energy range and each pixel in the region of interest, inherent transmission characteristics of the material to the X-rays are calculated as inherent information at each of the pixels.

As a form of this inherent information, in Patent Document 2, for example, a three-dimensional liner attenuation vector ($\mu_1 t$, $\mu_2 t$, $\mu_3 t$) is calculated. Specifically, the X-ray attenuation amount $\mu_i t$ ($\mu$: linear attenuation coefficients in the X-ray path direction, t: a length of the X-ray path, where i = 1, 2, 3) is calculated for each of the three energy ranges (energy BIN) and at each pixel. Based on the X-ray attenuation amount $\mu_i t$, the above-mentioned three-dimensional linear attenuation vectors ($\mu_1 t$, $\mu_2 t$, $\mu_3 t$) are calculated. The 3D attenuation vectors ($\mu_1 t$, $\mu_2 t$, $\mu_3 t$) are further normalized to a fixed length. This results in the calculation of the 3D mass attenuation vectors ($\mu_1'$, $\mu_2'$, $\mu 3'$) which have no relation to any factor of thickness t or density.

The 3D mass attenuation vectors ($\mu_1'$, $\mu_2'$, $\mu_3'$) reflect the degree of X-ray attenuation due to the elements which are present in the path of the X-ray path projected on each pixel. Therefore, the 3D mass attenuation vectors ($\mu_1'$, $\mu_2'$, $\mu_3'$) extending from the coordinate origin have a meaningful 3D direction. In other words, this 3D direction indicates the direction specific to a substance (that is, one element or a combination of several elements) that is present in the X-ray path on the 3D coordinate. In other words, this vector direction will be the same for the same material or material composition. Therefore, if a noise factor is excluded, the vector directions theoretically exist as identical 3D mass attenuation vectors. Accordingly, it can be analyzed how the scattered points of the location of the tip of the 3D mass attenuation

vector ($\mu_1$', $\mu_2$', $\mu_3$') for each pixel on the normalized 3D map are spread. This analysis can provide information on the type and properties of such elements (substances).

[Citation List]

[Patent Literature]

[PTL 1] JP 2013-119000 A
[PTL 2] WO 2016/171186 A1

[Non-Patent Literature]

[Non-PTL 1] Anritsu Technical No.87, Mar. 2012, "Development of Dual-Energy X-Ray Inspection System"

[Summary of the Invention]

[Technical Problem]

The analysis of the scatter of the 3D mass attenuation vectors ($\mu$1', $\mu$2', $\mu$3' ($\mu$: linear attenuation coefficient)) described in the foregoing document 2 is as follows. Specifically, in this analysis, the scattered points on the normalized 3D map are collectively sorted. This sorting is performed by grouping, that is, for each set that can be considered to be a scatter point of a mixture of the same one or more elements. This group of scattered points is further determined by its center of gravity. A 3D vector connecting the center of gravity and the coordinate origin is adopted as the 3D mass attenuation vector representing such a group.

<Loss of length information>

According to the analysis method exemplified in this patent document 2, there is still a drawback. Even though the 3D linear attenuation vectors ($\mu_1$t, $\mu_2$t, $\mu_3$t) have information reflecting the factor of lengths t, it is still necessary to normalize the length t of the vectors. This means that the length information is lost in the process, and therefore the data indicating the amount of the substance cannot be obtained from the analysis results. In other words, in the case of the analysis method shown in this patent document 2, the best the skilled person can do is to estimate the type of substance.

<Problems due to large computation amounts, which results in necessity for unit region>

Furthermore, in the case of the 3D scatter diagram proposed in Patent Document 2 described above, the number of scatter points to be plotted on the diagram is usually extremely large and plotted in analog amount. For this reason, the computational load on the processor is very large to calculate the plotted data as they are.

Because of these factors, it is necessary to significantly reduce the amount of computation in analyzing material identification information from scattered points, even when there are many scattered points, without using the length information of the 3D mass attenuation vector ($\mu_1$', $\mu_2$', $\mu_3$') for each pixel. Hence, it is necessary to provide an X-ray system that is easy to install in actual clinical settings and factories.

The present invention has been made in consideration of the situation faced by the conventional X-ray inspection as described above. In other words, the objective is to "provide information on the type and properties of a substance that constitutes the entirety or a part of an inspection target (region of interest) by X-rays, and information indicating the amount of the substance (consisting of one element or a combination of multiple elements)" with "a smaller amount of calculation and with higher accuracy". An apparatus and method for processing X-ray detection data that can act for achieving the foregoing objective, and an X-ray inspection device equipped with the apparatus, are provided.

[Solution to Problem]

In order to realize the foregoing object, in the present invention, there is provided a data processing device in which continuous X-rays are irradiated to an object, the continuous X-rays having "n" different energy ranges (n is a positive integer of 3 or more), and data indicating an attenuation degree of the X-rays which have been transmitted through the object are processed at each of detector pixels of a detector for each of the n different energy ranges. The data processing device is provided with pixel vector calculating means, which are configured to calculate an n-dimensional vector corresponding to the n piece based on the data, the n-dimensional vector indicating a linear attenuation value when the X-rays of each of the n different energy ranges are transmitted through the object; representative vector calculating means, which are configured to mutually add, for each of a plurality of search regions virtually set based on one or more detector pixels of the plurality of detector pixels, the n-dimensional vectors of the detector pixels which respectively belong to the plurality of search regions in an n-dimensional space, thereby calculating, for every one of the search regions, an n-dimensional representative vector representing each of the search regions; and substance information acquiring means, which are configured to acquire, from an amount, type, and property of the substance in the object, at least the amount and the type of the substance, based on both the representative vector for each of the search regions and a desired-size unit region which is virtually set in a substance space defined as having, as coordinate information, degrees of attenuation of the X-rays which are transmitted through the object.

According to this configuration, the representative vector is calculated for each search region. In this calculation, the n-dimensional vectors of the detector pixels belonging to the search region are vector-added, that is, the components are added in the direction of each n-dimensional axis to obtain a representative vector that represents the whole. Therefore, it is possible to reflect the direction of the n-dimensional vectors obtained for each detector pixel and to perform processing for acquiring material information based on the representative vector by reflecting the length component of each n-dimensional vector as it is.

Moreover, the unit region of a desired size, which is virtually set in the material space with the coordinate information of the degree of attenuation of the X-ray when the X-ray penetrates the object, is used to acquire the material information. In other words, the representative vector on the n-dimensional coordinate space is replaced by the material space that has the degree of attenuation of X-rays as coordinate information, and the material information is obtained for each unit region on the material space. Therefore, by setting the size of the unit region to an arbitrary size, the material information of many representative vectors in the n-dimensional coordinate space can be classified in the material space (so to speak, digitized into multiple values).

In this way, without losing the length information of the representative vectors in each search region, and by classifying the region into unit regions in the material space, we can make use of the information on the amount (thickness) of the material in the object that we originally had. This makes it possible to obtain material information that at least reflects the quantity of material. This is a groundbreaking feature because it was previously thought to be difficult. If the search regions are in different positions and the unit region on the same material space as the other search regions is the same, then the amount of each substance can be related. This also makes it possible to determine the amount of substance in each material region across all search regions.

In addition, the material space can be defined, and material information can be analyzed with the length information of representative vectors for each unit region in this material space.

Therefore, information indicating the amount of material in the object can be provided with less computation and with higher accuracy.

This data calculation can be implemented not only as a data processing device as described above, but also as a data processing method with a similar calculation function. Furthermore, it can be similarly implemented as an X-ray inspection system (medical X-ray diagnostic device, non-destructive X-ray device, etc.) in which such a data processing device or data processing method is installed as an integrated unit or as a unit linked by communication.

[Brief Description of the Drawings]

In the accompanying drawings:

Fig. 1 is a block diagram outlining an X-ray inspection apparatus of one embodiment to which a data processing apparatus and a data processing method according to the present invention is applied;
Fig. 2 is a graph explaining a relationship between a continuous X-ray spectrum and energy bins which are set in the spectrum:
Fig. 3 is an illustration explaining detector pixels and three-dimensional vector at the respective pixels, which are obtained by photon counting detection of an energy discrimination type;
Fig. 4 is a flow diagram outlining procedures to obtain the three-dimensional vectors shown in Fig. 3;
Fig. 5 is a functional block diagram showing the basic configuration of the present invention;
Fig. 6 is a block diagram detailing vector addition, which is related to a part of the basic configuration of the present invention;
Fig. 7 is a pictorial illustration showing part of the basic configuration of the present invention;
Fig. 8 is a functional block diagram detailing obtaining substance information, which is a part of the basic configuration of the present invention;
Fig. 9 is a pictorial illustration of mesh regions (unit regions) which are set on an argument coordinate, which is a part of the basic configuration of the present invention;

Fig. 10 is an illustration exemplifying how to set the mesh regions (unit regions);
Fig. 11 is an illustration showing an example of acquisition of substance information;
Fig. 12 is an illustration showing another example of acquisition of substance information;
Fig. 13 is an illustration showing a part of a tooth row of a subject being tested, which is modeled from a viewpoint of substances, the tooth row being adopted here as an object for explaining another example of acquisition of substance information; and
Fig. 14 is an illustration exemplifying acquisition of a true three-dimensional representative vector of hard tissue which is based on the model of the part of the tooth row shown in Fig. 13.

[Detailed Description of Embodiments of the Invention]

Hereinafter, with reference to the accompanying drawings, an embodiment of a data processing device and a data processing method which are according to the present invention, and an X-ray system equipped with the data processing device or implemented with the data processing method will now be described.

<Embodiment>

Referring to Figs. 1-14, a data processing device and a data processing method which are according to one embodiment will now be described.
The data processing device and method are applicable to an X-ray system in which continuous X-rays (also referred to as polychrome X-rays) having a continuous energy spectrum are irradiated to an object being inspected, and the intensities of the transmitted X-rays are detected by an X-ray detector.
In particular, this data processing device and method are based on photon-counting X-ray detection, with which the number of photons of irradiated X-rays is counted as X-ray intensity information. This detection is suitably applicable to X-ray systems (such as medical X-ray diagnostic apparatuses and non-destructive X-ray inspection apparatuses) that perform X-ray detection while discriminating each of X-ray photons into any of a plurality of predetermined energy bins.
There are two energy discrimination methods, which are follows. One method is to discriminate at the receiver side by using threshold values for received signals which are set in the X-ray detector. The other method, which is still equivalent to the former in terms of energy discrimination, is an energy discrimination method with which only X-rays with energies belonging to two or more specific energy ranges predetermined in the X-ray generator are irradiated. For the latter, it is known to combine one or more X-ray filters that block or transmit specific wavelength X-rays in advance, or to use multiple X-ray tubes with different target materials. A typical example of the latter is known as the DEXA method.
Thus, the data processing method and the data processing device according to the present embodiment are applicable to any system that uses energy discrimination type X-ray detection. Especially, when photon-counting X-ray detection is equipped, this detection gives a system maximized accuracy. Specific examples of such systems include an X-ray mammography apparatus, a dental X-ray apparatus, and other medical X-ray apparatuses, as well as an X-ray apparatus for foreign matter inspection. Especially when being equipped with photon-counting X-ray detection, the accuracy is maximized in such systems.
When the data processing device and method of this embodiment according to the present embodiment are applied to such X-ray apparatuses, the device and method may be implemented or mounted as an integral part of the system, or may be implemented or mounted on a terminal remotely networked to those systems by communication lines. Furthermore, X-ray transmission data detected by a system that performs energy-discriminated photon-counting X-ray detection may be implemented or mounted on a stand-alone processing unit.
Such a system for energy-discriminated photon-counting X-ray detection is known from International Publication Number WO2015/111728 A1 and others.
The basic configuration of the X-ray system is shown in Fig. 1. As shown in the figure, the X-ray system (X-ray device) 10 is equipped with an X-ray generator 21 that generates continuous spectrum X-rays, collimates the X-rays into a beam, and irradiates the object space OS. The X-ray generator 21 is equipped with an X-ray tube 22 driven by a high voltage supply, and a collimator 23 located at the output side of the X-ray tube 22, which collimates the X-rays generated by the X-ray tube 22 into a beam of X-rays. The focal diameter of the tube focus F of the X-ray tube 22 is, for example, 0.5 mmcp. The X-ray tube 22 has a tube focal point F which can be regarded as an almost point X-ray source. The X-rays emitted from the tube focal point F consist of a flux of photons with various energies (X-ray energy amounts), and have a continuous energy spectrum depending on the tube voltage.
The X-ray system 10 is further equipped with a detector 24 that detects the beam of X-rays that have been irradiated and transmitted through the object OB located in the object space OS. The detector 24 has a detection layer 25 made of a semiconductor (such as CdTe or CZT) that directly converts incident X-rays into corresponding electrical signals. The detection layer 25 is positioned directly below the detector incident window. A group of pixels is formed in this detector layer 25, which is formed into, for example, a two-dimensional array of pixels having a size of 200 μm x

200 μm.

Of course, what is called, an indirect conversion type X-ray detector can be used instead of the foregoing direct conversion type semiconductor detector, if a system is equipped with a photon counting type detection configuration described later. In this indirect conversion type detector, X-rays are converted into optical signals by a scintillator, and then, these optical signals are converted into electrical signals by a semiconductor device.

The detector 24 also has a layered data acquisition circuit 26 below the detection layer 25, which processes the detection signal of each pixel on a pixel-by-pixel basis. This circuit 26 is built in an ASIC, for example. This data acquisition circuit 26 is configured as a photon counting type circuit that can count the number of photons of X-rays incident on a group of pixels in the detection layer 25 for each pixel. In addition, this circuit sets a threshold for discriminating X-ray energy. This setting divides the X-ray spectrum into multiple X-ray energy ranges (called BINs). Therefore, it is possible to count photons for each pixel in each of those energy BINs.

Therefore, the counting data is output from the layered data acquisition circuit 26 as frame data (a set of counting data for each pixel). This frame data is created by processing the electrical pulse signals in response to the incident X-ray photons for each pixel at each energy BIN. The frame rate varies from 300 fps to 6,600 fps, and as an example. except for the superposition phenomenon of photons incident on one pixel, for example, one photon incident excites one electric pulse. Therefore, the counting data for each pixel reflects the number of electric pulses.

As mentioned above, this detector is classified as a photon counting type detector in terms of the detection process. In other words, in this detector, X-rays with a continuous energy spectrum (polychromatic X-rays) are regarded as a set of photons with various energies. Under such a view, the detector 24 is configured to count the number of these photons by X-ray energy BIN (range) and pixel by pixel (there may be one or more pixels). As shown in Fig. 2, for example, three energy BIN: Bini to $Bin_3$ are set as the energy BIN. The number of these energies BIN: Bin can be four or five, as long as it is more than three. The ranges below the lower threshold TH1 and above the upper threshold TH4 (equivalent to the tube voltage) of the energy [keV] are the ranges that cannot be measured or are not used. Therefore, this range between the threshold values TH1 and TH4 is divided into one (in this case, the threshold values are only TH1 and TH4) or multiple energy BINs. For example, the threshold values TH2 and TH3 are set as shown in Figure 2, and three energy BINs are formed.

The above configuration of X-ray irradiation and detection has been proposed by International Publication Number WO2015/111728 A1 and others.

The imaging object OB located in the object space OS is scanned by a beam of X-rays. For this purpose, it is sufficient to have a configuration in which one of the X-ray generator 21 and detector 24 pairs and the OB to be photographed moves relative to the other. As an example, in the case of X-ray inspection of foreign objects such as food, a belt conveyor is arranged to pass through the object space OS. By placing the OB to be photographed on this conveyor belt, the OB to be photographed is scanned with X-rays. Another example of a medical system is a dental panoramic radiography system. In this system, the patient's jaw, which is the OB to be photographed, is positioned in the object space OS formed between the X-ray generator 21 and the detector 24. In this state, the pair of X-ray generator 21 and detector 24 are rotated while facing each other, and the jaw is thus scanned with X-rays. The same configuration is used in an X-ray mammography system as an example of a medical system. In short, it is sufficient that the OB to be photographed is scanned with relative movement between the X-ray generator 21 and detector 24 pairs and the OB to be photographed.

The digital amount of measurement data output from the detector 24 is sent to a processing unit mounted on the X-ray system 10 or to a processing unit located outside the X-ray system 10. This processing device performs processing that takes advantage of the energy discrimination. This processing includes image reconstruction using the tomosynthesis method, creation of an absorption vector length image (2-D image) based on the reconstructed image, and creation of a 3-D scatter plot based on the reconstructed image. These processes have been proposed by International Publication Number WO2016/171186 A1 and others.

The X-ray system 10 of this embodiment is equipped with a data processing device 30. As shown in Fig. 1, this data processing device 30 is composed of a microcomputer CP, as an example. This computer CP itself can be configured as a computer with known arithmetic functions. The computer CP is equipped with an interface (I/O) 31 that is connected to the detector 24 via a communication line LN. This interface 31 has a buffer memory 32, a ROM (read-only memory) 33, a RAM (random access memory) 34, a CPU (central processing unit) 35A via an internal bus B processor 35, image memory 36, input device 37, and display device 38. These elements are communicatively connected to each other via a bus B. The name of the processor 35 may be referred to as an arithmetic unit, an arithmetic unit, or the like. In addition, a Micro-Processing Unit (MPU) may be used instead of a CPU. Of course, ROM and RAM as memory can also be used in a variety of known forms.

Various programs for computer-readable measurement value correction and substance identification, etc. are stored in advance in ROM 33. For this purpose, the ROM 33 has a storage region 33A (which functions as a non-transitory computer recording medium) to store those programs in advance. In addition, this ROM 33 also has first and second storage regions 33B, 33C for storing beam hardening correction data (also called calibration data) for beam hardening correction of measured values.

The processor 35 (i.e., CPU 35A) reads the necessary program from the storage region 33A of ROM 33 into its own work region and executes it. The processor 35 is a CPU for image processing. The buffer memory 32 is used to temporarily store frame data sent from the detector 24. The RAM 34 is used to temporarily store the data necessary for the calculation during the calculation of the processor 35.

The image memory 36 is used to store various image data and information processed by the processor 35. The input device 37 and the display device 38 function as a man-machine interface with the user. Of these, the input device 37 accepts input information from the user. The display unit 38 is capable of displaying images and other information under the control of the processor 35.

The data processing device 30 may be provided as a diagnostic device or inspection device integrated with the X-ray system 10, as described above. The data processing device 30 may be communicatively connected to the X-ray system 10 via a communication line LN, as in the present embodiment. In such a case, it may be connected online at all times, or it may be communicable only when necessary. Furthermore, the data processing device 30 may be provided in a stand-alone format. Of course, the data processing device 30 may consist of a hardware circuit that performs pipeline processing, etc.

<Data collection and data acquisition>

Therefore, according to the X-ray system 10 described above, the attenuation values $\mu_1$, $\mu_2$, and $\mu_3$ based on the linear attenuation factors $\mu_1$, $\mu_2$, and $\mu_3$ corresponding to the three energies BIN: Bini, $Bin_2$, and $Bin_3$ described above are calculated for each of the pixels P1, P2, P3, ..., and Pn of the detector 24, as shown schematically in Fig. 3. The attenuations $\mu_1 t$, $\mu_2 t$, and $\mu_3 t$ are calculated based on the linear attenuation coefficients $\mu_1$, $\mu_2$, and $\mu_3$ corresponding to the three energies BIN: Bini, $Bin_2$, and $Bin_3$ described above. Here, t is the length (thickness) of the path of the X-ray beam through the object. In other words, these three physical amounts, the attenuation $\mu_1 t$, $\mu_2 t$, and $\mu_3 t$, are each taken as one physical dimension. This allows us to build up a three-dimensional vector of linear attenuation coefficients ($\mu 1t$, $\mu 2t$, $\mu_3 t$) at each pixel P1 (P2, P3, ..., Pn) by calculation. Part of Fig. 3 shows this 3-dimensional vector ($\mu_1 t$, $\mu_2 t$, $\mu_3 t$) schematically. This 3-dimensional vector ($\mu_1 t$, $\mu_2 t$, $\mu_3 t$) can also be referred to as the pixel vector in this form.

Moreover, in each detector pixel, there is a substance consisting of one or more elements in the object that exists along the x-ray path projected toward that pixel (hereinafter referred to simply as the substance). Therefore, the three-dimensional gradients ($\theta$, $\varphi$: see Fig. 3) of its three-dimensional vectors ($\mu_1 t$, $\mu_2 t$, $\mu_3 t$) have been found to be unique to that substance. In other words, except for variations due to statistical noise, the three-dimensional gradients ($\theta$, $\varphi$) have the same value depending on the type of element that constitutes the material. This has been shown in International Publication No. WO2016/171186 A1 (3D scatter plot) and other publications.

Furthermore, the length L of the 3D vectors ($\mu_1 t$, $\mu_2 t$, $\mu_3 t$) represents a physical amount that reflects information about the amount of X-ray attenuation incident on each detector pixel. In other words, the larger the linear attenuation coefficient $\mu$, and (and/or) the larger the thickness t of the material, the larger the X-ray attenuation amount $\mu t$. This is likewise shown by International Publication No. WO2016/171186 A1 (3D scatter plot) and others.

For this reason, in this embodiment, as the raw data necessary to perform the given specific material identification process, the data processing unit 30 receives the photon counts output from the detector for each of its pixels, i.e., the number of outgoing photons $Co_1$, $Co_2$, $Co_3$ (Fig. 4, Step S11). Furthermore, the number of incident photons to each detector pixel is calculated as $CI_1$, $CI_2$, $CI_3$, and the number of outgoing photons $Co_1$, $Co_2$, $Co_3$, and

$$Co_1=CI1e(-\mu_1 t)$$
$$Co_2=CI2e(-\mu_2 t)$$
$$Co_3=CI3e(-\mu_3 t)$$

(where e denotes the exponential function), calculate the X-ray attenuation amounts $\mu_1 t$, $\mu_2 t$, and $\mu_3 t$ corresponding to the three energies BIN: Bini, $Bin_2$, and $Bin_3$, respectively (Fig. 4, Step S12).

In this explanation, $\mu_1$, $\mu_2$, and $\mu_3$ are the hypothetical average linear attenuation coefficients at each energy BIN: Bini to $Bin_3$ (i.e., linear attenuation coefficients for the effective energy of each energy BIN). It is assumed that these $\mu_1$, $\mu_2$, and $\mu_3$ are independent of the thickness t. The incident photon counts $CI_1$, $CI_2$, and $CI_3$ are the data collected without the object in place. This incident photon number is usually collected in advance.

Therefore, the data processing unit 30 calculates and stores a three-dimensional vector ($\mu_1 t$, $\mu_2 t$, $\mu_3 t$) for each detection pixel element based on the above-mentioned X-ray attenuation amounts $\mu_1 t$, $\mu_2 t$, $\mu_3 t$ (Fig. 4, steps S13 and S14). The process up to this point is also shown, for example, in International Publication No. WO2016/171186 A1. For this reason, the data processing device 30 functionally has pixel vector calculation means for calculating such three-dimensional vectors ($\mu 1t$, $\mu_2 t$, $\mu_3 t$), i.e., pixel vectors.

<The unique substance identification of this embodiment>

Once the three-dimensional vectors ($\mu_1$t, $\mu_2$t, $\mu_3$t) are ready as described above, the data processing device 30 can perform the substance identification described below, for example, in response to an interactive command from the user.

Here, substance identification refers to the identification of information indicating the amount of a substance in addition to information indicating the type of element (one or more elements) constituting the substance (e.g., effective atomic number Zeff) and/or information indicating changes in the properties of the substance. In this form, the ability to present this information indicating the amount of the substance is a breakthrough that was not possible in the past. In the past, it has already been shown that it is possible to present information that can identify the type of substance (a mixture consisting of one or more combinations of elements) in an object that has been irradiated with X-rays, and to present information that indicates changes in the properties of the substance. Here, the type of substance refers to distinguishing, for example, whether it is soft tissue or hard tissue, fat, calcium, or iron. The change in properties refers to the extent to which the substance has changed from its original state of elemental proportions, and so on. This change in properties includes, for example, the decomposition of a substance due to chemical actions such as oxidation and reduction. However, this alone is insufficient for substance identification these days, and there is a great need to obtain information on the amount of a substance in addition to information on its type and properties.

In this system, in addition to obtaining information on the type and (and/or) properties of substances, information on the amount of substances present along the X-ray path is also estimated with high accuracy. In other words, it is possible to obtain quantitative information on substances that are solid or scattered in the object. As a result, it is possible to obtain innovative information on substance identification based on X-rays, which was not previously possible, and to enrich the information.

For this purpose, the data processing unit 30 performs, in outline, representative vector calculation (Fig. 5, step S31) and substance information acquisition (step S32). (Of these, step S31 provides a representative vector calculation unit that functionally constitutes the representative vector calculation method, and step S32 provides a substance information acquisition unit that functionally constitutes the substance information acquisition method.

Among these, the representative vector operation targets each of the multiple search regions (or areas) (bundled detector pixels or one unbundled detector pixel) that are virtually set up based on one or more detector pixels in the multiple detector pixels. That is, the n-dimensional (e.g., three-dimensional) vectors of the detector pixels belonging to each of the said plurality of search pixels are mutually vector-added in the n-dimensional space to calculate the n-dimensional representative vector that represents each of the plurality of search regions. In other words, this representative vector is calculated for each search region (area).

After this representative vector calculation, the material information acquisition process is performed. In this material information acquisition process, information indicating at least one of the amount, type, and properties of the target material is acquired based on the representative vectors for each search region (area) and a unit region (area) of the desired size (also called a mesh region (area)) virtually set in the material space. Here, the material space is defined as a space that has as coordinate information the degree of attenuation of said X-rays when they penetrate said material.

This representative vector calculation (Step S31) and the acquisition of material information (Step S32) are described in detail in turn below.

<Representative vector calculation (Step S31)>

As shown in Fig. 7(A), four pixels $P_{11}$, $P_{12}$, $P_{21}$, $P_{22}$ that are adjacent to each other as pixels (detector pixels) physically set in the detector 24 are used as an example. The output signals from these four pixels $P_{11}$, $P_{12}$, $P_{21}$, $P_{22}$ are combined together and set as one search region $EX_{n, m}$ (n = 1, 2, ... p, m = 1, 2, ... q: p and q are positive integers of 2 or more) (Step S311). Of course, each pixel $P_{11}$ (~$P_{22}$) may be assumed to be one search region EX1.

For each of these four detector pixels $P_{11}$, $P_{12}$, $P_{21}$, $P_{22}$, three-dimensional vectors ($\mu_1$t, $\mu_2$t, $\mu_3$t) have been set by the preparation process described above, as shown in Fig. 3. Therefore, the data processing unit 30 reads the data of those three-dimensional vectors ($\mu_1$t, $\mu_2$t, $\mu_3$t) into the work region (Step S312).

Next, the data processing unit 30 performs vector addition (step S313), where the 3D vectors ($\mu_1$t, $\mu_2$t, $\mu_3$t) of each of the four detector pixels $P_{11}$, $P_{12}$, $P_{21}$, $P_{22}$ read out are mutually added to each other for each component of their three axes. As a result, the composite vector Vs of the search region $EX_{n,m}$ is obtained as a representative vector (see Fig. 7). This gives a vector amount that calculates the directional components of the three-dimensional vectors ($\mu_1$t, $\mu_2$t, $\mu_3$t) that each pixel $P_{i, j}$ (i, j = 1, 2) has.

Next, the data processing unit 30 calculates and stores the length VL of the composite vector Vs and its two argument angles $\theta$ and $\pi/2$-$\varphi$ (Step S314). This provides information on the length and argument angles that can uniquely define the composite vector Vs in three-dimensional space.

These processes are performed in order for all search regions $EX_{n, m}$ on the detector pixels (steps S315, S316).

<Acquisition of substance information (Step S32)>

Next, the data processor 30 performs the process of obtaining information on the substances that have been set for identification (Step S32 (Fig. 8: Steps S321 to S327)).

First, a two-dimensional argument coordinate is set by the data processing unit 30 (step S321: functionally corresponds to the two-dimensional argument coordinate setting method). These two-dimensional argument coordinates are two-dimensional coordinates that assign two argument angles $\theta$ and $\pi/2$-$\varphi$, respectively, that the representative vector makes with each of the two axes on the aforementioned three-dimensional coordinates. These two-dimensional argument coordinates are shown schematically in Fig. 9.

Next, a plurality of two-dimensional unit regions UR (n/2 - $\varphi_p$, $\theta_l$: p, k are positive integers and p = 1, ..., m, ..., l = 1, ..., n, ...) are virtually set (Step S322: functionally corresponds to the unit region setting method). This two-dimensional plurality of unit regions UR corresponds to the identification resolution when identifying the type of substance.

In setting the unit region, the data processing device 30 may display the two-dimensional argument coordinates in the process of setting on the display 38 and interactively adjust the size of the unit region UR ($\pi/2$-$\varphi_p$, $\theta_l$) with the user. The data processing unit 30 may also display the unit region UR ($\pi/2$-$\varphi_p$, $\theta_l$) of a predetermined default size (step S322A: functionally equivalent to the unit region display method).

This unit region UR ($\pi/2$-$\varphi_p$, $\theta_l$) is illustrated by the setting method based on Fig. 10(A), (B). This unit region UR ($\pi/2$-$\varphi_p$, $\theta_l$) may be set interactively with the user in the memory space by the data processing unit 30. Furthermore, the data processing unit 30 may set the unit region UR in memory in advance and read it out to the work region.

The unit region UR ($\pi/2$-$\varphi_p$, $\theta_l$) illustrated in Fig. 10(A) is set in memory (arithmetically) in advance or at the time when the system is used, in two-dimensional declination coordinates with $\pi/2$-$\varphi$ on the horizontal axis and $\theta$ on the vertical axis. The vertical axis is divided at equal pitch $P_\varphi$, and the horizontal axis is divided at the same or different pitch Pe. As a result, a square-shaped or rectangular unit region UR ($\pi/2$-$\varphi_p$, $\theta_l$) with equally spaced vertical and horizontal pitches is set in two-dimensional declination coordinates. It is assumed that a plurality of representative vectors Vs belong to each of these unit regions UR($\pi/2$-$\varphi_p$, $\theta_l$). If this assumption holds, then the materials on the X-ray path incident to the original detector pixels exhibiting those representative vectors Vs can both be regarded as materials consisting of the same element (in terms of effective atomic number Zeff). Therefore, this unit region UR (n/2 - $\varphi_p$, $\theta_l$) can determine whether the substances can be regarded as being of the same type or not. In other words, this process functions as a discriminator to determine whether or not the substance is the same for each search region $EX_{n,}$ m, which bundles detector pixels. Moreover, the directionality of the intrinsic vectors of the substance is represented by the unit region UR (n/2 - $\varphi_p$, $\theta_l$) on the two-dimensional declination coordinate. This representation significantly reduces the memory region to be used and the computational load, and speeds up the computation compared to the case where the vector is represented for each detector pixel.

In the unit region UR ($\pi/2$-$\varphi_p$, $\theta_l$) illustrated in Fig. 10(B), as the value on the horizontal axis: $\pi/2$-$\varphi$ increases, the division pitch $P_\varphi$ gradually increases, and as the vertical axis: $\theta$ increases, the division pitch Pe gradually as the value on the vertical axis: $\theta$ becomes larger. Here, we assume that, for example, substances with atomic numbers Z = 7 to 13 are represented on these declination coordinates. In the case of this assumption, the distribution positions of the declination angles $\pi/2$-$\varphi_p$ and $\theta$ of these substances do not necessarily follow a straight line. In other words, as shown for reference in Figs. 10(A) and 10(B), it can be said to be a straight line when the atomic number is small, but the interval between each scattering point becomes narrower and more curved as the atomic number increases. Therefore, in the case of the setting method of the unit region in Fig. 10(B), the division pitch Pe is gradually narrowed in consideration of the fact that the spacing between the scatter points becomes narrower as the atomic number becomes larger. In this way, even when the atomic number becomes narrower, the accuracy of discriminating whether or not a substance is of the same type (including cases where it can be regarded as substantially the same type of substance) is not reduced. Incidentally, substances with atomic numbers Z = 7-13 regions an example, multiple types of substances that exist in the oral cavity.

This unit region UR (n/2 - $\varphi_p$, $\theta_l$) is not necessarily limited to setting by dividing horizontally and vertically on the two-dimensional argument coordinate. On the same coordinates, it may be divided by diagonal lines for each axis, or by using curves. This unit region is to gather one or more 3D representative vectors Vs that indicate the same kind of substance into the same unit region as much as possible, and to calculate the weight value on the substance space described below as accurately as possible. For this reason, a suitable division region can be set in relation to this accuracy.

Next, two argument angles ($\pi/2$-$\varphi$, $\theta$) of the 3D representative vectors (composite vectors) Vs for each of the aforementioned search regions $EX_{n, m}$ are calculated by the data processing unit 30 (Step S323: functionally equivalent to argument angle calculation method).

After this argument calculation, a judgment is made as to which unit region UR ($\pi/2$-$\varphi_p$, $\theta_l$) the two argument angles ($\pi/2$-$\varphi$, $\theta$) of the three-dimensional representative vectors Vs in the two-dimensional argument coordinates are classified into (belong to), based on the values of the argument angles. (Step S324: Functionally called argument determination method). For example, if there are multiple 3D representative vectors Vs having the same or similar values of argument ($\pi/2$-$\varphi$, $\theta$), they are judged to belong to the same unit region UR ($\pi/2$-$\varphi_m$, $\theta_n$) (see Fig. 9). The result of the judgment is recorded.

Next, for each unit region UR (n/2 - $\varphi_p$, $\theta_l$), the data processing unit 30 reads out the 3D representative vectors Vs of each of the plurality of search regions $EX_{n,}$ m belonging to the unit region in the work region. Furthermore, the vector lengths of those 3D representative vectors Vs area added to each other as weight values (step S325: functionally equivalent to the weight value adding method). This weight value is a scalar amount expressed as weight value = $\mu_m\rho t$, where $\mu_m$ is the mass attenuation coefficient of the material presenting the 3D representative vectors Vs grouped in each unit region UR (n/2 - $\varphi_p$, $\theta_l$), $\rho$ is the density of the material, and t is the thickness (amount) of the material on the X-ray path. The weight value = $\mu_m'\rho t$ represents the degree of X-ray attenuation in each unit region UR ($\pi/2$-$\varphi_p$, $\theta_l$) and reflects the information t of the amount of the substance.

Next, a three-dimensional or two-dimensional weight value image is calculated for each unit region UR ($\pi/2$-$\varphi_p$, $\theta_l$), with the added weight value as the pixel value. Furthermore, the image data is recorded (step S326: functionally corresponds to the weight value image calculation means).

In other words, the data processing device 30 can record the position information of each virtually combined search region in the material space and the pixel values consisting of weight values in correspondence with each other as one process of acquiring material information, pertaining to the recording method. This allows weight value images to be generated in a ready manner and such images to be displayed in response to a demand easily.

A part (A) of Fig. 11 schematically shows an example of a three-dimensional weight value image, where the weight value "$\mu_m'\rho t$" is assigned to the two-dimensional argument ($\pi/2$-$\varphi$, $\theta$) as the height dimension. This mass attenuation coefficient $\mu_m$ can be defined as a representative mass attenuation coefficient representing each unit region UR ($\pi/2$-$\varphi_p$, $\theta_l$). This representative mass attenuation coefficient $\mu_m'$ is

$$\mu_m' = \{(W_1\mu_{1m})^2 + \ldots + W_3\mu_{3m})^2\}^{1/2}$$

can be expressed as W1 to W3, which correspond to the energy regions BIN: Bini to $Bin_3$, are weight coefficients and can be taken as appropriate values. In (A) of Fig. 11, a phantom or a virtual phantom with atomic number Z=7 to 13 is used as an object for reference. For this reason, the weight value "$\mu_m'\rho t$" of the material with atomic number Z = 7, 8, 9, ..., 13 is expressed as height information, that is, information reflecting the amount of the material.

Therefore, for example, using a phantom or a virtual phantom with atomic number Z=7-13, a reference curve with the atomic number on the horizontal axis and the representative mass attenuation coefficient $\mu$m' on the vertical axis can be obtained as shown in (B) of Fig. 11. In this case, this reference curve is used to calibrate the distribution position of the three-dimensional weight value image shown in (A) of Fig. 11, and the weight value "$\mu_m'\rho t$" is divided by the representative mass attenuation factor $\mu_m'$. This results in a 3D material image that shows the information of the amount of material "ρt" independent of the attenuation coefficient shown in (C) of Fig. 11. According to this 3D material image in (C) of Fig. 11, as the atomic number increases and the argument angles $\pi/2$-$\varphi$ and $\theta$ become larger, the magnitude of the amount of material "ρt" also increases. However, the increase is suppressed, so the 3D material image is easy to see.

Furthermore, this 3D material image in (C) of Fig. 11 is projected onto a 2D plane with a argument angle of $\pi/2$-$\varphi$, $\theta$, and its height information is expressed, for example, in terms of brightness and color. This results in the two-dimensional material image shown in (D) of Fig. 11.

The parts (A), (B) and (C) of Fig. 11 are coordinate spaces that contain information on the type and amount of substance, respectively, as well as the specific distribution of that substance. Therefore, these coordinates are also referred to as three-dimensional or two-dimensional substance space.

In the case of the parts (A) to (D) of Fig. 11 described above, it was assumed that the X-ray data taken from a phantom with atomic numbers Z = 7 to 13 were processed. In reality, however, there are no such continuous objects. Therefore, the distribution will be obtained according to the atomic number and effective atomic number formed by one or more elements of the material contained in the object.

In addition, in various inspections, if a means is used to measure and understand the material composition of the target inspection object in advance, it can be expressed as the material information desired according to the purpose of the inspection.

Furthermore, information indicating at least one of the type, property, and amount of the substance is analyzed by the data processing device 30 based on the weight value image and the added value of the weight value $\mu_m\rho t$ (Step S327: Analysis means). In this analysis, in particular, information indicating at least the amount and type among the amount, type, and property of the substance of the object is obtained. One form of this analysis includes displaying that acquired information, for example, via a display unit 38.

This analysis can also be provided in a wide variety of ways.

<Comparison method>

This analysis is a method in which, for example, the material image of the two-dimensional material space of the object obtained in Fig. 11(d) above is compared with a material image showing changes over time or a predetermined reference material image. Furthermore, in this method, the observed elements are more clearly depicted as a result of the comparison. In this case, the objects to be inspected must be imaged under the same imaging conditions. In other words, the same imaging conditions mean that the inspection object is under the same imaging conditions, such as the same X-ray tube voltage, the same positional relationship with the X-ray tube, and the same magnification ratio.

An example of this comparison method is shown schematically in Fig. 12. In this figure, a reference image $A_{original}$ taken in the patient's mouth is prepared, and a material image $B_{original}$ taken for comparison is prepared.

These images $A_{original}$ and $B_{original}$ are smoothed by, for example, a Gaussian filter or a Blur filter to obtain the filtered images $A_{filter}$ and $B_{filter}$. Then, for each pixel of these images $A_{filter}$ and $B_{filter}$, the operation of $A_{filter}/B_{filter}$ is executed to obtain the comparison image $A_{filter}/B_{filter}$.

With this comparison image $A_{filter}/B_{filter}$, the material image captured can be compared with reference images or standard (average) material distributions from databases with various viewpoints, without the amount of material being dependent on density or mass attenuation factor. This allows the differences resulting from the comparison to depict changes over time more prominently. In addition, if reference images of a standard phantom of "soft tissue + bone tissue" and/or a standard phantom of bone tissue only are prepared in advance, the comparison difference with these reference images can be taken. In this way, as much soft tissue as possible can be removed from the "soft tissue + bone tissue" image, and as a result, the bone tissue to be seen can be depicted with higher accuracy.

<Separation method>

The following explains the method of separating soft and hard tissues from each other using the representative vectors Vs of each search region EXn, m by the separation method. As an example, we take a partial image of the dentition from intraoral radiography as an example, and explain this method with reference to Figs. 13 and 14. In this case, the data processing unit 30 also functions as a substance separation means to perform the separation process.

As shown in Fig. 13, the partial X-ray image of the dentition can be roughly divided into three parts: B1: the enamel part (classified as bone tissue), B2: the "dentition part (bone tissue) + alveolar bone (bone tissue) + soft tissue" part, and B3: the "alveolar bone (bone tissue) + soft tissue" part. Moreover, the values of the effective atomic number Zeff of bone tissue in parts B1, B2, and B3 are different from each other. On the other hand, even if we assume that the soft tissues in parts B2 and B3 both have the same or similar effective atomic number Zeff, it will cause little error in the analysis. This is because the length of the representative vector Vs of the soft tissue is much smaller than that of the hard tissue.

Therefore, the following method is adopted to separate the hard tissue from this partial image of the dentition from intraoral radiography.

First, the directions of the representative vectors Vss of the search regions EXn and m for only the soft tissues of the mouth structure are created in advance as a reference database. In creating this reference database, the beam hardening phenomenon of the X-ray acquisition signal is corrected for each detector pixel by using a phantom that is an artificial material similar to the soft tissue using a known method. The representative vector Vs is calculated based on the corrected signal.

In other words, as an example, the data processing device 30 obtains correction data for beam hardening correction of X-rays based on a phantom having an effective atomic number similar to that of a specific target material (i.e., the device 30 functions as a means of obtaining correction data). Furthermore, the data processing device 30 can apply beam hardening correction to the subtracted data based on the correction data (i.e., the device 30 functions as a correction means). As a result, the aforementioned pixel vector is calculated for each detector pixel as an n-dimensional vector based on the corrected data.

Next, the region in the mouth is imaged. In addition, as described above, the representative vectors Vs for each search region $EX_{n,m}$ are calculated by the data processing unit 30, and these are temporarily stored.

This calculated representative vector Vs is represented by the longer vector extending from the origin in the 3D coordinate of the amount of attenuation shown in Fig. 14. In other words, it is a composite vector for bone tissue and soft tissue, i.e., it is represented as vector information that has been actually imaged and calculated. In this figure, the shorter representative vector Vss extending from the origin is the representative vector for the reference soft tissue only, which was collected in advance as described above.

Then, this representative vector Vss for reference is read out from the database by the data processing unit 30. Then, for example, as described above, the scatter points pre-collected using the phantom showing atomic number Z = 7, 8, 9, ... 13 are mapped on the 3D attenuation coordinates. Then, a curve CV that smoothly connects these scatter points is calculated and mapped.

After this, the data processing unit 30 calculates the plane PL (see shaded region) connecting the above curve CV, the length direction of the composite vector Vs corresponding to the actual image capture, and the origin.

Next, a line is drawn from the tip of the above curve CV and the composite vector Vs along the above plane PL and in the direction of the representative vector Vss of the soft tissue. The intersection point KT of this line and the curve CV is calculated. In other words, from the measured representative vectors Vs corresponding to bone and soft tissues, the representative vectors Vss for soft tissues, which are held in advance as reference, are vector-subtracted in the 3D subtraction space (which is also a kind of material space). In this way, the actual representative vector Vsb is calculated for the bone tissue only. At this time, the tip of this representative vector Vsb is located on the above curve CV, and its position indicates the effective atomic number Zeff of the substance presenting the representative vector Vsb.

Therefore, the data processing unit 30 repeats the above calculation for all search regions $EX_{n, m}$. This allows the effective atomic number Zeff and the representative vector Vsb, which indicate the bone tissue existing in the partially photographed region of the mouth, to be collected in all search regions $EX_{n, m}$ respectively. The information of this representative vector Vsb can be processed into a three-dimensional or two-dimensional material image in the same way as described above. In addition, the effective atomic number Zeff can be displayed in various ways, such as superimposing it on a partial image. Furthermore, the change in the effective atomic number Zeff can be deciphered. These types of analysis can be used to more accurately detect changes in bone density.

This is due to the fact that, when performing the 3D vector subtraction of "bone tissue + soft tissue" - "soft tissue", the calculation is not a simple vector subtraction in space. In other words, the phantom with the reference atomic number is used, and the beam-hardened surface of this phantom (i.e., the surface PL formed by the curve CV above) is used. In addition, the vector subtraction is performed according to this plane. This means that the direction and length of the representative vector Vsb for the obtained bone tissue can be estimated with higher accuracy. In other words, the degree of the beam hardening phenomenon on multiple kinds of materials differs depending on the atomic number. Taking this into account, this system can estimate the change in the state of bone tissue (bone density, etc.) and the information reflecting the amount of the change with higher accuracy.

The information indicating the direction of the representative vector Vsb for the reference soft tissue may be obtained by using actual patient's collected data when performing the above calculation.

In addition, when the data processing of the above form is performed, the attenuation caused by the air layer may be subtracted from the detected X-ray transmission data as a pre-processing step. It is desirable to perform the above substance identification using the collected data in each energy region, which has eliminated the noise caused by the air layer as much as possible. This pre-processing is provided as a functionally provided air layer subtraction method by the data processing unit 30.

In addition, although not specifically detailed, it is necessary to perform beam hardening correction on the above collected data using artifactual materials with compositional composition similar to that of one or more specific imaging objects.

Furthermore, it is desirable to multiply the X-ray attenuation values $\mu 1t$ to $\mu 3t$ obtained in both detector pixel units and energy region units by the weight factors of each W1 to W3.

As described above, in the configuration of the data processing device, the data processing method, and the X-ray system of the present embodiment, the representative vector is calculated for each search region. In this calculation, the n-dimensional vectors of the detector pixels belonging to each search region are vector-added, that is, the representative vectors respectively representing the whole search regions are obtained by adding the components in each n-dimensional axis direction. Therefore, a representative vector is obtained that reflects the direction of the n-dimensional vector calculated for each detector pixel and the length component of each n-dimensional vector. Based on this representative vector, processing for material information acquisition can be performed.

Furthermore, the unit region of the desired size, which is virtually set in the material space, is used to acquire the material information. This material space has the coordinate information of the degree of attenuation of the X-rays when the X-rays penetrate an object being examined. In other words, the representative vector on the n-dimensional coordinate space is replaced by the above material space, and the material information is obtained for each unit region on the material space. For this reason, the size of this unit region can be set to any arbitrary size. By this arbitrary setting, the material information of many representative vectors in the n-dimensional coordinate space can be classified in the material space (that is, digitized into multiple values).

In this way, without losing the length information of the representative vectors in each search region, and by classifying the region into unit regions in the material space, users can make use of the information on the amount (thickness) of the material in the object that we originally had. This makes it possible to obtain material information that at least reflects the amount of material. This is a groundbreaking feature because obtaining this material information was previously thought to be difficult. Here, it can be assumed that the search regions are in different locations and the unit region on the same material space as the other search regions is the same. If this is the case, then the amount of each substance can be treated in the same way. In this way, the entire search region can be treated in the same way. This also allows us to understand the amount of substance in each material region across all search regions.

In addition, the foregoing material space can be defined, and the material information can be analyzed and displayed with the length information of representative vectors for each unit region in this material space.

Therefore, information indicating the amount of material in the object can be provided with less computation and with higher accuracy.

Various further variations are possible in the above embodiment, as follows.

In some cases, the substance to be tested can be regarded as consisting of essentially two characteristic substances. In such a case, a phantom with an effective atomic number similar to those two specific target substances can be set up and corrected.

The system may be configured to weight the beam hardening corrected data with a weighting factor for each of the three energy regions as a plurality of energy regions and for each detector pixel. The weighting factor may be set to maximize the signal-to-noise ratio, which is the amount of noise relative to the signal, of the representative vector determined from the three energy regions.

As another form of analysis and display, the system may be equipped with means to generate and display an image from the weighted value image in which the pixel values are the amounts corresponding only to the density $\rho$ of the material and the thickness t in the transmission path direction in the material of the X-rays.

Furthermore, as another form of analysis and display, a representative vector length image can be generated from the representative vector, where the length of the representative vector is the pixel value for each search region. In this case, a means can be provided to reconstruct the pixel values of detector pixels based on the data from the detector to recreate the original image. In that case, furthermore, a region of interest may be set in one of at least two images, the original image representative vector length image and the weighted value image, as another form of analysis and display. In that case, information indicating the location of the set region of interest may be further displayed on the other image in conjunction with the location of the region of interest.

As another way of analysis and display, a region of interest may be set on one of the representative vector length image and the weighted value image. In that case, information indicating the location of the region of interest set in one of the images may be displayed on the other image in conjunction with the location of the region of interest. In that case, furthermore, the local image data of the representative vector length image and the weighted value image that form a part of the region of interest may be stored.

In addition, an image processing method may be provided to further process the local image data.

In this X-ray system, as an example, the two specific target substances region bone-equivalent substance and soft tissue.

Furthermore, in the X-ray system of this embodiment, the n is an integer of two or more, and the continuous X-rays belonging to each of said integer energy regions are irradiated to the object in chronological order from one X-ray generator, or are irradiated to the object individually and in chronological order from an integer number of X-ray generators. The data indicating the degree of attenuation of the X-rays may be data indicating the integral value of the energy of X-ray photons per unit time incident on each of the detector pixels for a certain period of time or the total value of such X-ray photons for a certain period of time, and may be data output from an integral type or photon counting type X-ray detection device.

In this X-ray system, n is an integer of two or more, and the X-rays irradiated from one X-ray generator to an object and transmitted through the object are outputted to the X-ray detectors of the X-ray integrating type or the X-ray photon counting type, which are arranged in order from the near side to the far side from the X-ray generator, respectively. The data processing device as claimed in any one of claims 1 to 30, wherein the data corresponding to the continuous X-rays belonging to each of the integer energy regions is outputted from the integer number of X-ray detection devices in accordance with the detection characteristics of the integer energy regions.

<About the applications>

The present invention has a variety of features including the following. First, the contrast resolution of the image is high, and the sharpness is excellent. These advantages make it possible to simultaneously estimate the substance and information indicating the amount of substance, which has never been possible before. The estimation accuracy is very high. In addition, both can be grasped intuitively in the image, and imaging is possible even if the X-ray dose is the same level as that of diagnostic equipment currently used for imaging in various fields. Moreover, based on the principle of the present invention, the X-ray dose can be reduced to 1/3 to 1/4. Therefore, it can be widely used in the fields of medical equipment, non-destructive testing, and homeland security. Some of the possible applications are described below, along with a brief description of the effects.

<Medical applications>

(1) Mammography: Reduction of patient exposure dose, optimization of X-ray dose by identifying mammary gland content, and improvement of sensitivity for detecting malignant masses in higher mammary gland patients.
(2) Chest radiography system: Reduction of patient exposure dose, improvement of lung cancer detection rate by increasing sharpness and contrast resolution, detection of thyroid abnormality by iodine detection, detection of osteoporosis, detection of calcification, etc. as additional information.
(3) Orthopedic diagnostic equipment: Detection of early fracture, detection of rheumatism, detection of osteoporosis, implant planning, etc.
(4) Intraoral radiography equipment: Reduction of patient exposure dose, detection of osteoporosis, examination of periodontal disease, detection of inflammatory reaction, evaluation of pros and cons of implants, prognosis, etc.
(5) Dental panoramic system ... Patient dose reduction, osteoporosis diagnosis, periodontal disease examination, carotid artery calcification, maxillary sinusitis examination, etc.
(6) Contrast agent application: Reduction of patient exposure dose, significant reduction of contrast agent, functional diagnosis of heart, liver, kidney, etc., identification of targets, assisting diagnosis of healing degree, etc., combined use with therapeutic devices, treatment planning and cancer diagnosis by combination with heavy metal nanoparticle contrast agents.
(7) X-ray system for rounds: Reduction of patient exposure dose, downsizing, significant improvement in diagnostic accuracy, and increased flexibility in imaging.
(8) Medical and dental CT: Patient exposure dose reduction, significant improvement in accuracy over current Spectroscopic CT.
(9) Body composition analyzers: Reduction of patient exposure doses, downsizing, significant improvement in the accuracy of bone mineral determination, fat content analysis, etc., and application to sports medicine.
(10) X-ray systems for home use: Reduction of patient exposure dose, downsizing, highly accurate diagnosis in combination with telemedicine, which sends image information to a remote reader for diagnosis, medical care for developing countries, and reduction of diagnostic burden for base hospitals in developed countries.

<Non-destructive testing applications>

(1) Inspection of foreign objects in food: Downsizing of equipment, improvement of detection sensitivity, identification of foreign object types, and dramatic expansion of inspection targets.
(2) Bite inspection: High sensitivity, compact size, simultaneous inspection with foreign matter.
(3) Inspection systems that can be used in supermarkets and other retail locations: X-ray systems that can be used outside of X-ray controlled regions, and that can be used in locations close to retail locations (convenience stores, supermarkets, etc.), such as food foreign object inspection systems or spoilage detection systems.
(4) Compositional analysis of fresh fish and meat: Analysis of fat content, live inspection of fish and livestock, determination of muscle mass, parasite inspection, etc.
(5) Fracture inspection of racehorses: downsizing of inspection equipment, improvement of portability, inspection of early stage fractures, efficiency improvement by enlargement of inspection section, etc.
(6) Pearl cultivation inspection: Downsizing, speeding up, inspection of pearl growth and shape, etc.
(7) Inspection of changes in the properties of food products: looking at oxidation/reduction reactions in fermentation and putrefaction processes, etc.
(8) Analysis equipment for paintings, antiques, mural paintings of ancient tombs, etc.: component analysis of paints and mixed materials, etc.
(9) Lithium battery inspection equipment: Internal structural inspection, inspection of abnormal energy storage, etc.
(10) Industrial product inspection equipment: Missing product inspection, assembly error inspection, defective product inspection, thickness measurement, oil deterioration, etc.
(11) Metal product inspection: Inspection of internal defects, cracks, nests, corrosion, etc.
(12) Piping pipe inspection: Cracks, wall thickness, corrosion (oxidation) inspection, etc.
(13) Emulsion liquid inspection: Control of two-liquid mixing degree and liquid composition, etc.
(14) Distinction of multiple metals: Distinction and classification of mixed metals, inspection of specific metal content, inspection of metal purity, etc.

<Homeland security applications>

(1) Luggage inspection: Smaller device, higher detection accuracy, can be used in airports, museums, halls, customs, stadiums, public conference halls, etc.
(2) Narcotics and drug testing: High speed, high accuracy testing.
(3) Banknote counterfeit inspection: High accuracy, high speed inspection.
(4) Radioactive material inspection: Regular hazardous material inspection, simultaneous inspection of crops and fish for radioactive materials, soil inspection, etc.

The data processing device, data processing method, and X-ray system of the present invention are not limited to the configurations of the embodiments and variations described above. They can be further modified in various ways, such as by combining them with previously known configurations, to the extent that the abstracts described in the claims are not changed.

## Claims

1. A data processing device in which continuous X-rays are irradiated to an object, the continuous X-rays having n different energy ranges, where n is a positive integer of 3 or more, and data indicating an attenuation degree of the X-rays which have been transmitted through the object are processed at each of detector pixels of a detector for each of the n-piece energy ranges, wherein

    pixel vector calculating means, which is configured to calculate an n-dimensional vector corresponding to the n piece based on the data, the n-dimensional vector indicating a linear attenuation value when the X-rays of each of the n different energy ranges are transmitted through the object;
    representative vector calculating means, which is configured to mutually add, every each of a plurality of search regions virtually set based on one or more detector pixels of the plurality of detector pixels, the n-dimensional vectors of the detector pixels which respectively belong to the plurality of search regions in an n-dimensional space, thereby calculating, every one of the search regions, an n-dimensional representative vector representing each of the search regions; and
    substance information acquiring means, which is configured to acquire, from an amount, type, and property of the substance in the object, at least the amount and the type of the substance, based on both the representative vector for each of the search regions and a desired-size unit region which is virtually set in a substance space defined as having, as coordinate information, degrees of attenuation of the X-rays which are transmitted through the object.

2. The data processing device of claim 1, **characterized in that** each of the plurality of search regions is configured as an region provided by a single detector pixel among the plurality of detector pixels.

3. The data processing device of claim 1, **characterized in that** each of the plurality of search regions is configured as a region provided by virtually combining mutually-adjacent plural detector pixels of a predetermined number, among the plurality of detector pixels.

4. The data processing device of claim 1, **characterized in that** each of the plurality of search regions is configured by separated regions among the plurality of detector pixels.

5. The data processing device of claim 2 or 3, **characterized in that**

    the number "n" is 3,
    the pixel vector calculating means is configured to calculate, as the n-dimensional vector, a three-dimensional vector for each of the search regions, and
    the representative vector calculating means is configured to calculate, for each of the search regions, a three-dimensional representative vector which represents the unit region in a three-dimensional space,
    wherein the substance information acquiring means is configured to acquire information indicating at least one of the type and property of the substance based on both the three-dimensional vector for each of the search regions and the unit region.

6. The data processing device of claim 5, **characterized in that**

the substance information acquiring means comprise
two-dimensional argument coordinate setting means which sets a two-dimensional argument coordinate with two axes to which two arguments of the representative vector in the three-dimensional coordinate are respectively assigned, and
unit region setting means which sets virtually two-dimensional unit regions in the two-dimensional argument coordinate, as each of the plurality of unit regions, the two-dimensional unit regions corresponding to resolutions in identifying the type of the substance.

7. The data processing device of claim 6, **characterized in that** the device comprises unit region displaying means which displays the two-dimensional unit regions set by the unit region setting means interactively in setting thereof interactively or after the setting.

8. The data processing device of claim 6, **characterized in that** the substance information acquiring means comprise

argument calculating means which calculates the two arguments of the three-dimensional representative vector for each of the search regions,
argument determining means which determines that the two arguments of each of the three-dimensional representative vectors are classified into which of the unit regions in the two-dimensional argument coordinate,
weight vale adding means which mutually adds, every one of the unit regions, vector lengths of corresponding three-dimensional representative vectors among a plurality of the three-dimensional representative vectors in the search regions, the corresponding three-dimensional representative vectors belonging to the unit region, wherein the vector lengths are regarded as weight values for each of the unit regions, the weight values indicating a degree of X-ray attenuation in each of the unit regions and being defined as $\mu_m \rho t$, where $\mu_m$ is a mass attenuation coefficient of the substance which exists in an X-ray path when the X-rays are transmitted toward each of the detector pixels through the substance, $\rho$ is a density of the substance which exists in the X-ray path, and t is a length of the X-ray path in the substance,
weight value image calculating means which calculates a weight value image whose pixel values are provided by the weight values calculated for each of the unit regions, and
analysis means which analyzes at least one of the type and property of the substance based on the weight value image and added values of the weight values.

9. The data processing device of claim 8, **characterized in that** the analysis means is configured to specify an atomic number of one element included in the substance or an effective atomic number of a substance composed of two or more elements included in the substance, based on the pixel values of the weight value image.

10. The data processing device of claim 8 or 9, **characterized in that** the analysis means is configured to identify information showing an amount of a substance indicated by an effective atomic number corresponding to the substance, based on the added values of the weight vales.

11. The data processing device of claim 10, **characterized in that** the analysis means is configured to identify information reflecting the amount (t) of the substance indicated by the effective atomic number.

12. The data processing device of claim 11, **characterized in that** the analysis means is provided with means for obtaining a product of the mass attenuation coefficient ($\mu$m) and the density (p) of the substance for each of the unit regions and means for obtaining the amount (t) from the obtained product.

13. The data processing device of claim 11 or 12, **characterized in that** the analysis means is configured to display information reflecting the amount (t) or an amount processed from the amount (t) therein.

14. The data processing device of any one of claims 8 to 13, **characterized in that** the substance information acquiring means is configured to record, mutually correspondingly, positional information of, of the search regions, respective search regions which are virtually combined in the substance space and pixel values of the weight values.

15. The data processing device of any one of claims 6 to 14, **characterized in that**
the unit region setting means is configured to, as the plurality of unit regions, a plurality of two-dimensional small regions set in the two-dimensional argument coordinate, the two-dimensional small regions being defined by dividing each of the two axes of the two-dimensional argument coordinate at desired widths.

**16.** The data processing device of 15, **characterized in that** the desired widths on each of the two axes are predetermined and spaced to be equal.

**17.** The data processing device of 15, **characterized in that** the desired widths on each of the two axes are predetermined and spaced to be unequal.

**18.** The data processing device of claim 17, wherein widths of the unequal intervals are unequally spaced so as to equalize the number of 3D representative vectors classified in each of the plurality of unit regions in accordance with an unequally spaced mapping of argument information of a plurality of elements on the 2D argument coordinates.

**19.** The data processing device of any one of claims 5 to 18, **characterized in that** the device is provided with air layer subtraction means for subtracting, from the data, the data corresponding to X-ray attenuation of the air layer through which the X-rays have penetrated, and
the pixel vector calculation means is configured to calculate the n-dimensional vector for each detector pixel based on the subtracted data.

**20.** The data processing device of claim 19, **characterized in that**

the device is provided with correction data obtaining means for obtaining correction data to perform beam hardening correction of said X-rays based on a phantom having an effective atomic number similar to that of a specific target substance among said substances; and
correction means for applying the beam hardening correction to the subtracted data based on said correction data,
wherein the pixel vector calculation means is configured to calculate the n-dimensional vector for each detector pixel based on the corrected data.

**21.** A data processing device of claim 20, which sets up and corrects a phantom with an effective atomic number similar to that of the two specific target substances when the substances can be regarded as consisting of substantially two characteristic substances.

**22.** A data processing device of any one of claims 6 to 21, **characterized in that**
the analysis means is configured to separate, from one another, a plurality of substances composing the object by performing subtraction between the respective three-dimensional representative vectors for the plurality of substances in the three-dimensional coordinate.

**23.** The data processing device of claim 20, **characterized in that** the device is equipped with weighting means for weighting the data corrected by the correction means with a weighting factor for each of said three energy regions and for each detector pixel.

**24.** The data processing device of claim 23, **characterized in that** the weighting function is set such that the weighting coefficients are set so that the signal-to-noise ratio, which is an amount of noise relative to the signal of the representative vector determined from the three energy regions, is maximized.

**25.** The data processing device of claim 8, **characterized in that** the analysis means is provided with means to generate an image from the weighted value image in which the pixel values are the quantities corresponding only to the density $\rho$ of the substance and the thickness t in the transmission path direction of the X-ray in the substance.

**26.** The data processing device of claim 8, **characterized in that** the analysis means is provided with representative vector length generating means for generating a representative vector length image from the representative vectors, wherein the length of the representative vector is provided based on the pixel value for each of the search areas.

**27.** The data processing device of claim 26, **characterized in that**

the device is provided with original image recreating means recreating an original image by reconstructing the pixel values of the detector pixels based on the data, and
the analysis means is provided with

region-of-interest region setting means for setting a region of interest in one of at least two images among the original image, a representative vector length image, and the weighted value image; and
coordinated display means for displaying information indicating the position of the region of interest set in one of the images on the other image in coordination with a position of the region of interest.

**28.** The data processing device of claim 26, **characterized in that**

the analysis means comprises
region-of-interest setting means for setting a region of interest on one of the representative vector length image and the weighted value image;
coordinated display means for displaying information indicating a position of the region of interest set in one of the images in conjunction with a position of the region of interest in the other image; and
image data storage means for storing local image data of the representative vector length image and the weighted value image that form a portion of the region of interest.

**29.** The data processing device of claim 28, **characterized in that** the device comprises image processing means for further processing the local image data.

**30.** The data processing device of claim 21, **characterized in that** the two specific target substances are a bone-equivalent substance and soft tissue.

**31.** The data processing device of any one of claims 1 to 30, charactered in that

the "n" energy ranges are "n" energy regions set on a single spectrum of continuous X-rays, and
data indicating a degree of attenuation of the X-rays is data indicating a total number of X-ray photons per unit time incident on each of the detector pixels, and is data output from a photon-counting X-ray detection device.

**32.** The data processing device of any one of claims 1 to 30, charactered in that

the number "n" is an integer of two or more,
continuous X-rays belonging to each of integer energy ranges are irradiated to the object chronologically from one X-ray generator or individually and chronologically from the integer number of X-ray generators, and
data indicating a degree of attenuation of the X-rays is data indicating an integral value of energy of X-ray photons per unit time incident on each of the detector pixels for a certain period of time or a total value of the X-ray photons for a certain period of time, and is data output from an integral type or photon counting type of an X-ray detection device.

**33.** The data processing device of any one of claims 1 to 30, charactered in that

the number "n" is an integer of two or more, and
X-rays irradiated from one X-ray generator to the object and transmitted through the object are output to an X-ray integrating type or an X-ray photon counting type of X-ray detector for an integer number of X-ray detectors arranged in order from a near side to a far side from the X-ray generator, data corresponding to continuous X-rays belonging to each of integer energy ranges being output from the X-ray detectors in accordance with the detection characteristics of the integer energy ranges set respectively.

**34.** The data processing device of claim 22, **characterized in that**
the analysis means is configured to separate, from one the other, two types of substances of the object which are provided in the X-rays transmitted through the object, based on a plane formed by a three-dimensional representative vector indicating a composite substance of the two types of substances, an origin of the three-dimensional coordinate, and a curve formed by smoothly connecting scattered points on the three-dimensional coordinate, the scattered points being aligned when beam hardening correction is applied to a plurality of materials with different atomic numbers, the materials including the two types of materials.

**35.** The data processing device of claim 34, **characterized in that**,

in a case where one of the two types of substances is separated from the other type of material with reference to the other type of material,

the analysis means is configured to prepare, a known reference vector in advance, the three-dimensional representative vector of the other type of material, a projection is made on the plane from a tip of the three-dimensional representative vector representing the composite material to a surface along the direction of the reference vector, and obtain a vector connecting the origin and a projected point on the plane and the origin, as a true three-dimensional representative vector of the one type of material.

**36.** An X-ray system in which the data processing device according to any one of claims 1-34 is mounted as an integral part thereof or is separated and functionally linked thereto.

**37.** A data processing method of processing data treated in a photon counting X-ray detecting apparatus, wherein photons of X-rays having energies respectively belonging to "n" energy ranges (n is a positive integer of 3 or more) set on a spectrum of continuous X-rays enter respectively physical detector pixels whose number is preset, and counted in number per unit time, the data indicating a count of the photons, **characterized in that** the method including steps of:

Calculating an "n" dimensional vector corresponding to the "n" piece based on the data, the n-dimensional vector indicating a linear attenuation value when the X-rays of each of the "n" different energy ranges are transmitted through an object;
mutually adding, every each of a plurality of search regions virtually set based on one or more detector pixels of the plurality of detector pixels, the n-dimensional vectors of the detector pixels which respectively belong to the plurality of search regions in an n-dimensional space, thereby calculating, every one of the search regions, an n-dimensional representative vector representing each of the search regions; and
acquiring at least one from an amount, type, and property of the substance in the object, based on both the representative vector for each of the search regions and a desired-size unit region which is virtually set in a substance space defined as having, as coordinate information, degrees of attenuation of the X-rays which are transmitted through the object.

FIG.1

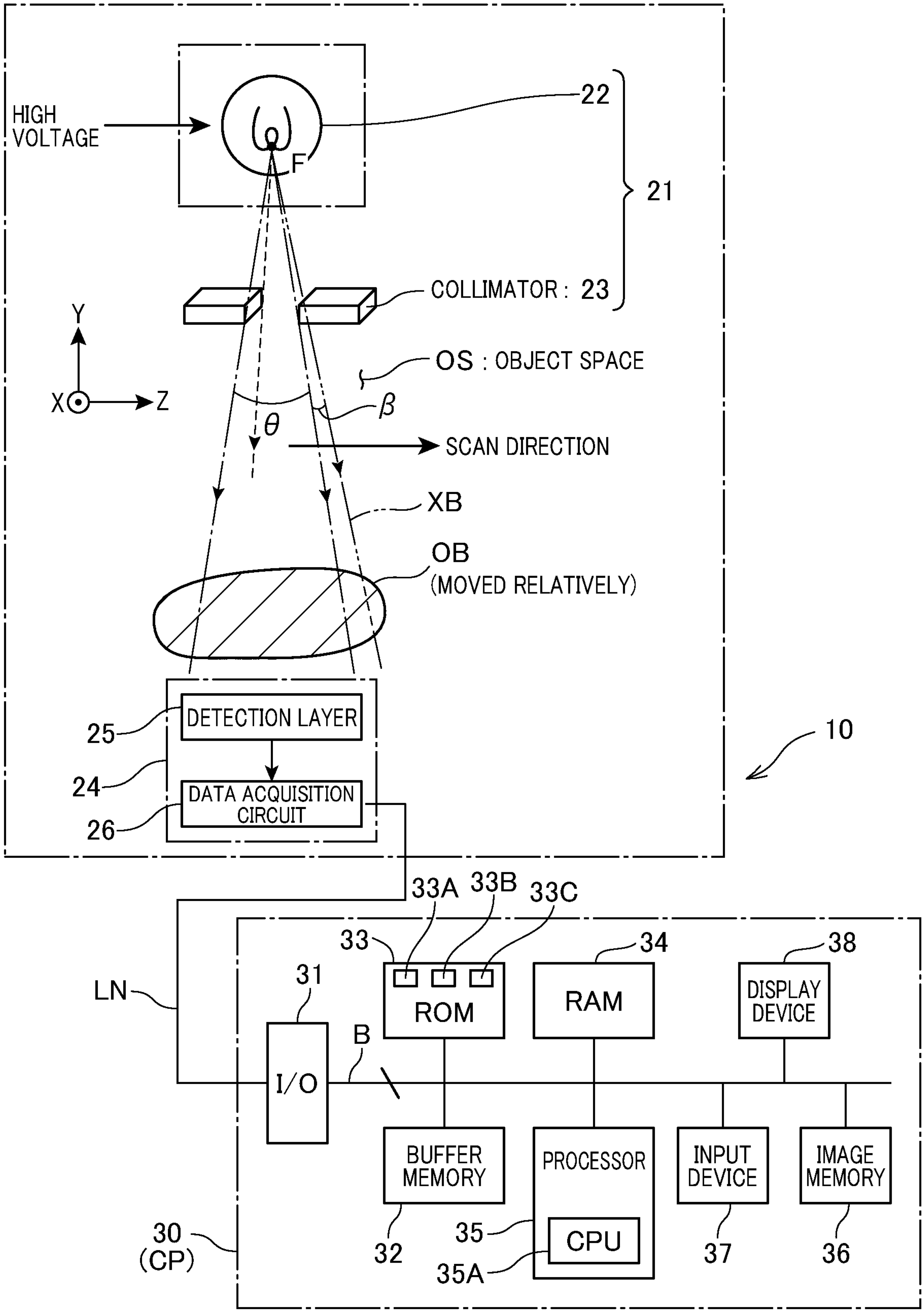
HIGH VOLTAGE
22
21
F
COLLIMATOR : 23
Y
X
Z
OS : OBJECT SPACE
θ
β
SCAN DIRECTION
XB
OB
(MOVED RELATIVELY)
25
DETECTION LAYER
24
DATA ACQUISITION CIRCUIT
26
10
33A
33B
33C
33
34
38
31
ROM
RAM
DISPLAY DEVICE
LN
B
I/O
BUFFER MEMORY
PROCESSOR
INPUT DEVICE
IMAGE MEMORY
30
(CP)
35
CPU
35A
32
37
36

FIG.2

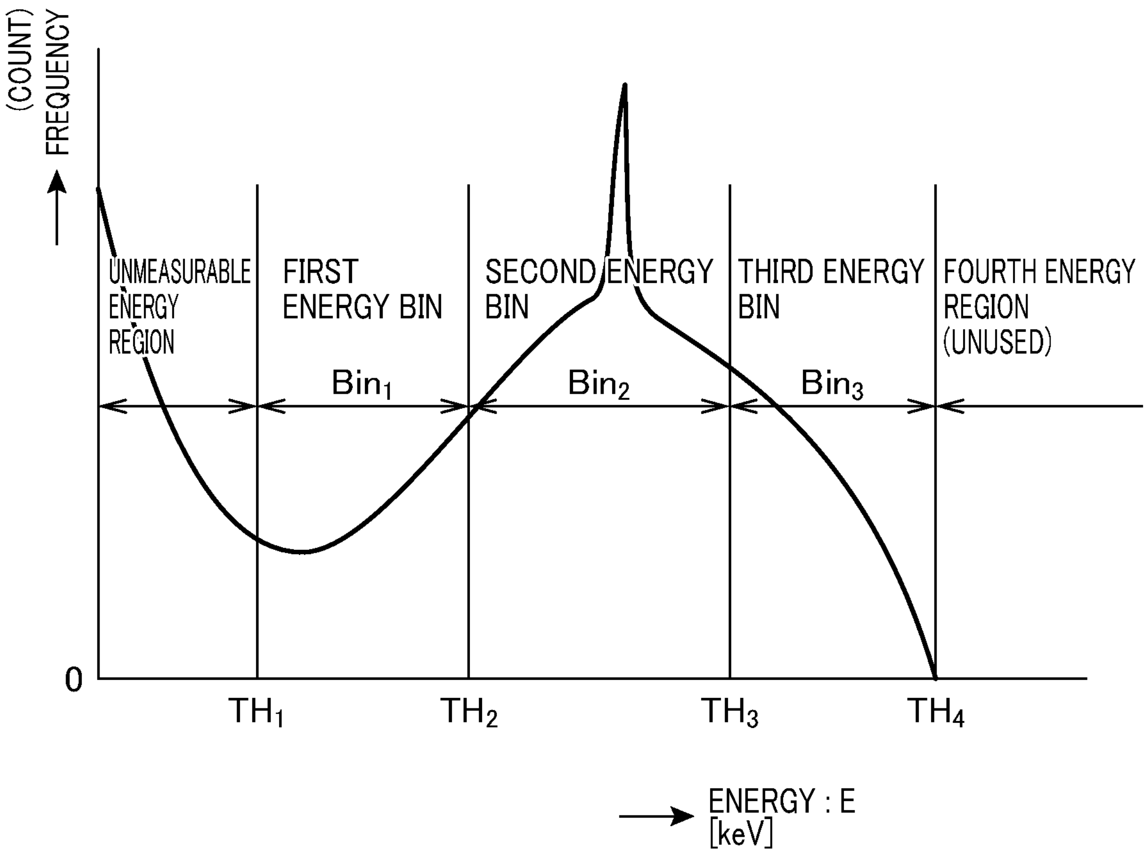
(COUNT)
FREQUENCY
UNMEASURABLE ENERGY REGION
FIRST ENERGY BIN
SECOND ENERGY BIN
THIRD ENERGY BIN
FOURTH ENERGY REGION (UNUSED)
Bin1
Bin2
Bin3
0
TH1
TH2
TH3
TH4
ENERGY : E [keV]

FIG.3

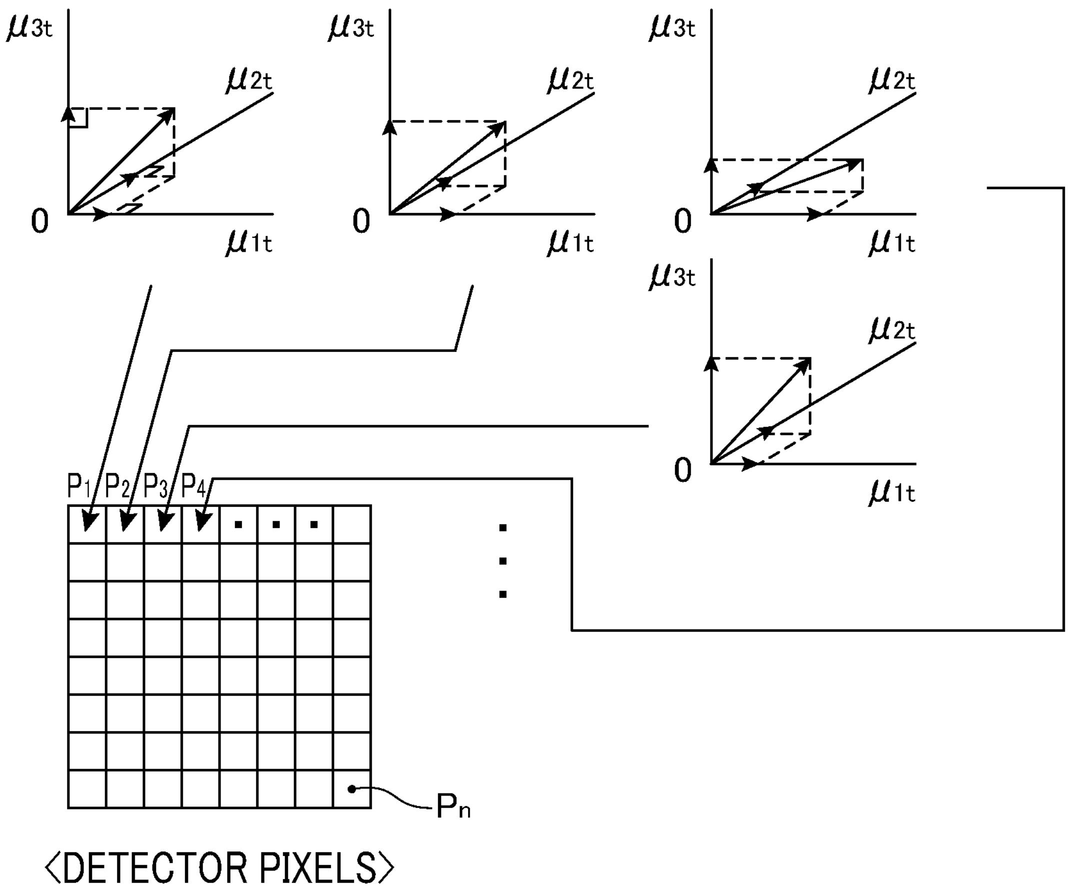

FIG.4

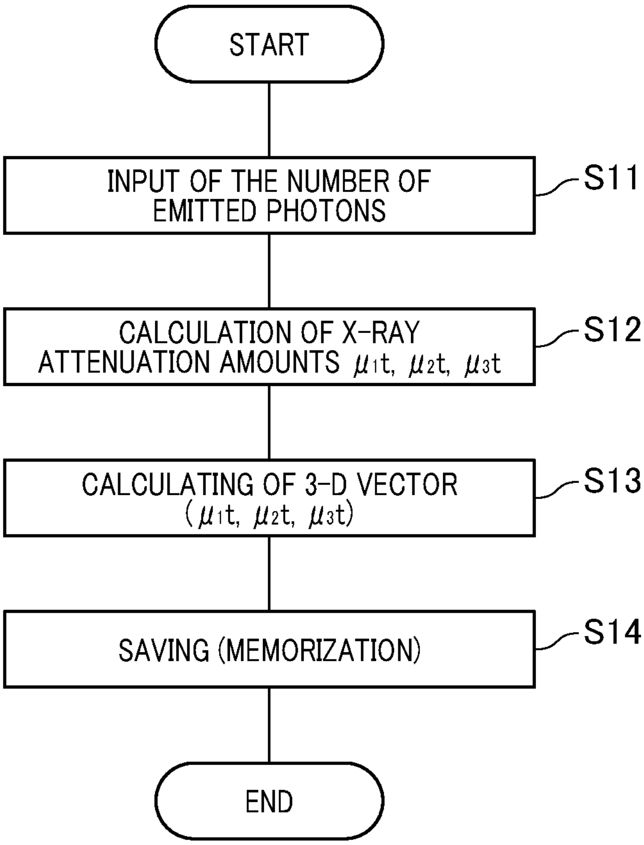
START
INPUT OF THE NUMBER OF EMITTED PHOTONS
S11
CALCULATION OF X-RAY ATTENUATION AMOUNTS $\mu_1t$, $\mu_2t$, $\mu_3t$
S12
CALCULATING OF 3-D VECTOR ($\mu_1t$, $\mu_2t$, $\mu_3t$)
S13
SAVING (MEMORIZATION)
S14
END

FIG.5

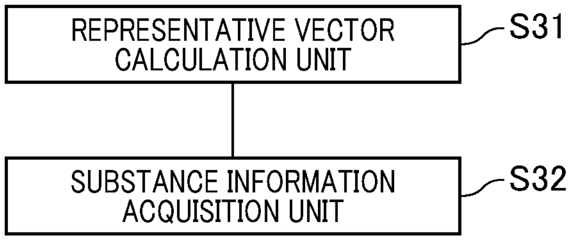
REPRESENTATIVE VECTOR CALCULATION UNIT
S31
SUBSTANCE INFORMATION ACQUISITION UNIT
S32

FIG.6

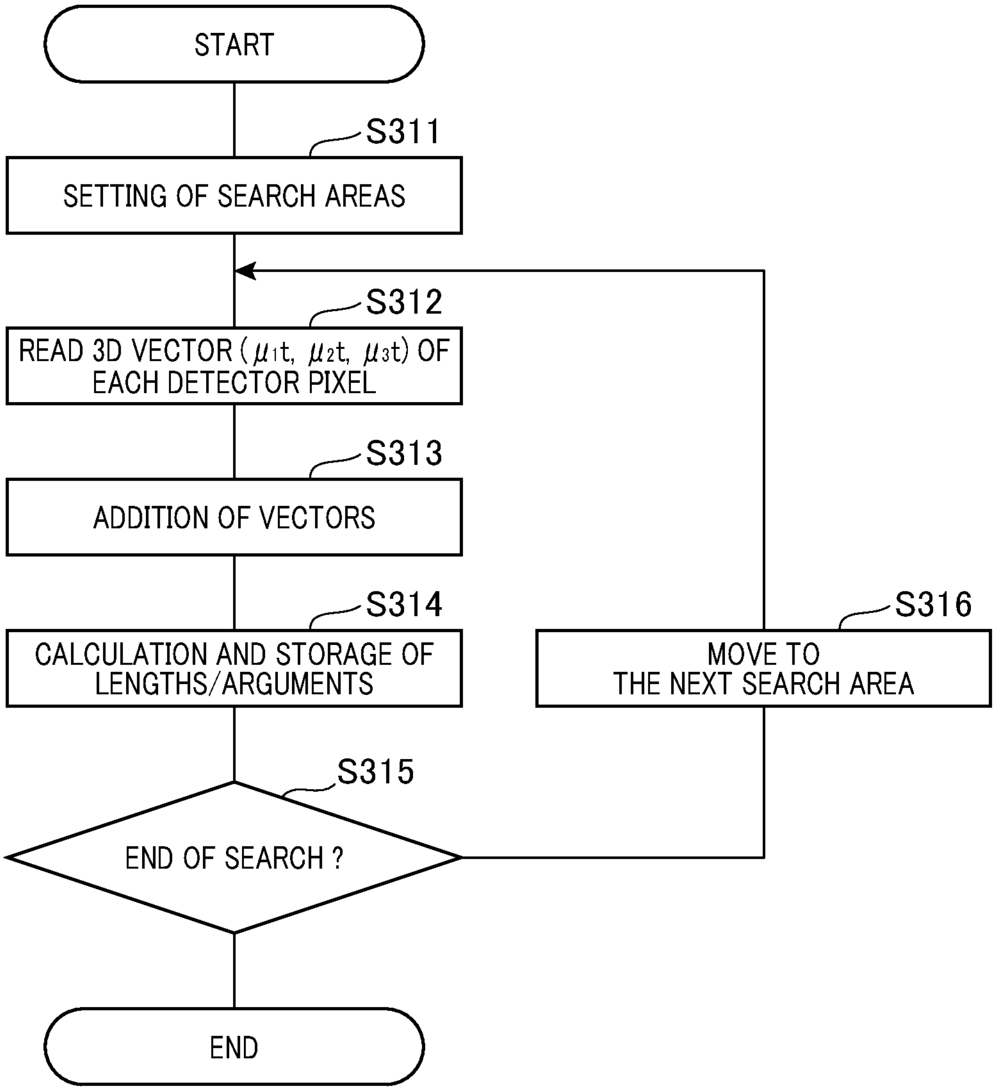
START
S311
SETTING OF SEARCH AREAS
S312
READ 3D VECTOR (μ1t, μ2t, μ3t) OF EACH DETECTOR PIXEL
S313
ADDITION OF VECTORS
S314
CALCULATION AND STORAGE OF LENGTHS/ARGUMENTS
S315
END OF SEARCH ?
S316
MOVE TO THE NEXT SEARCH AREA
END

FIG.7

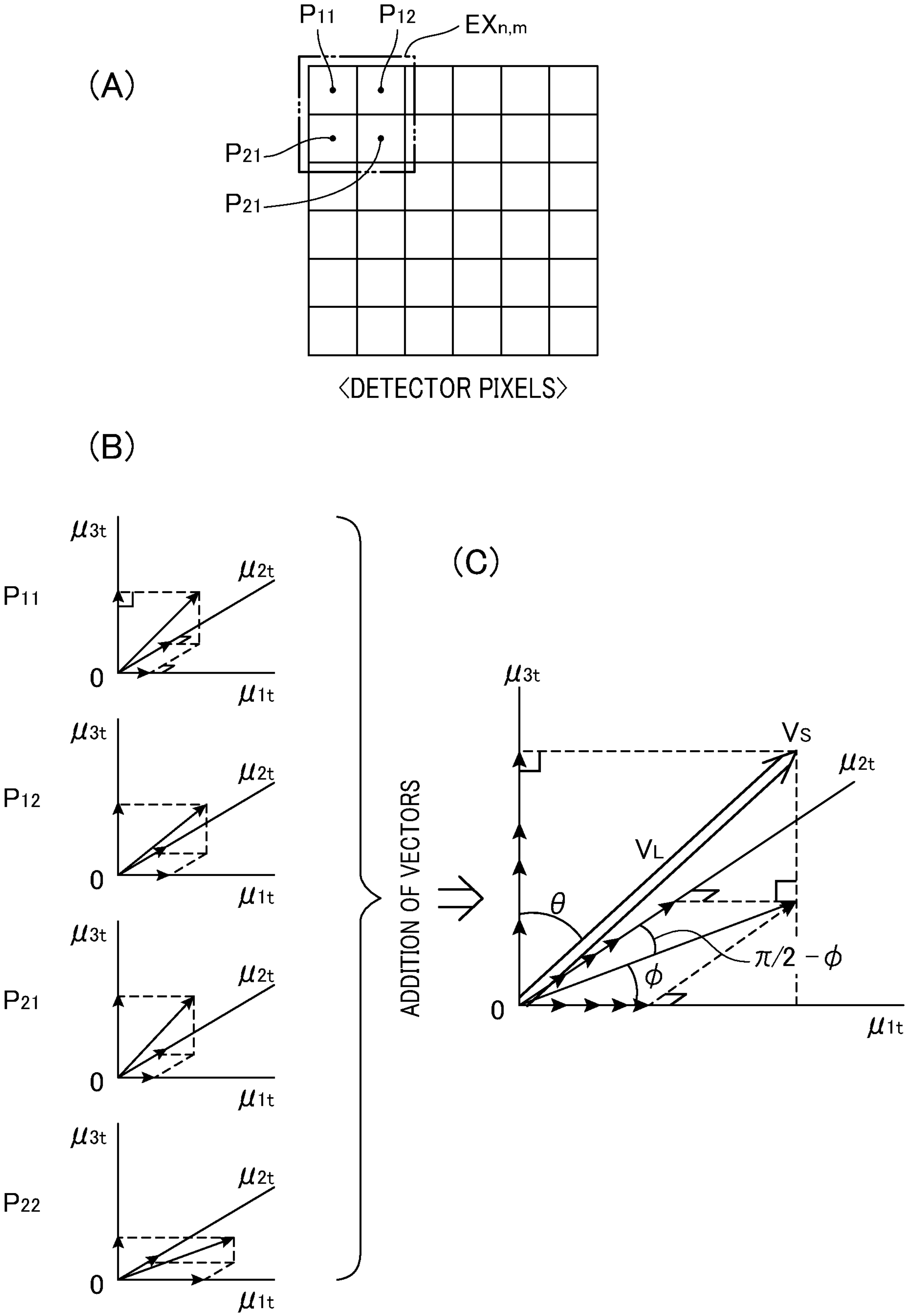
(A)
P11
P12
EXn,m
P21
P21
〈DETECTOR PIXELS〉
(B)
P11
P12
P21
P22
μ3t
μ2t
μ1t
0
ADDITION OF VECTORS
(C)
μ3t
VS
μ2t
VL
θ
π/2 − φ
φ
0
μ1t

FIG.8

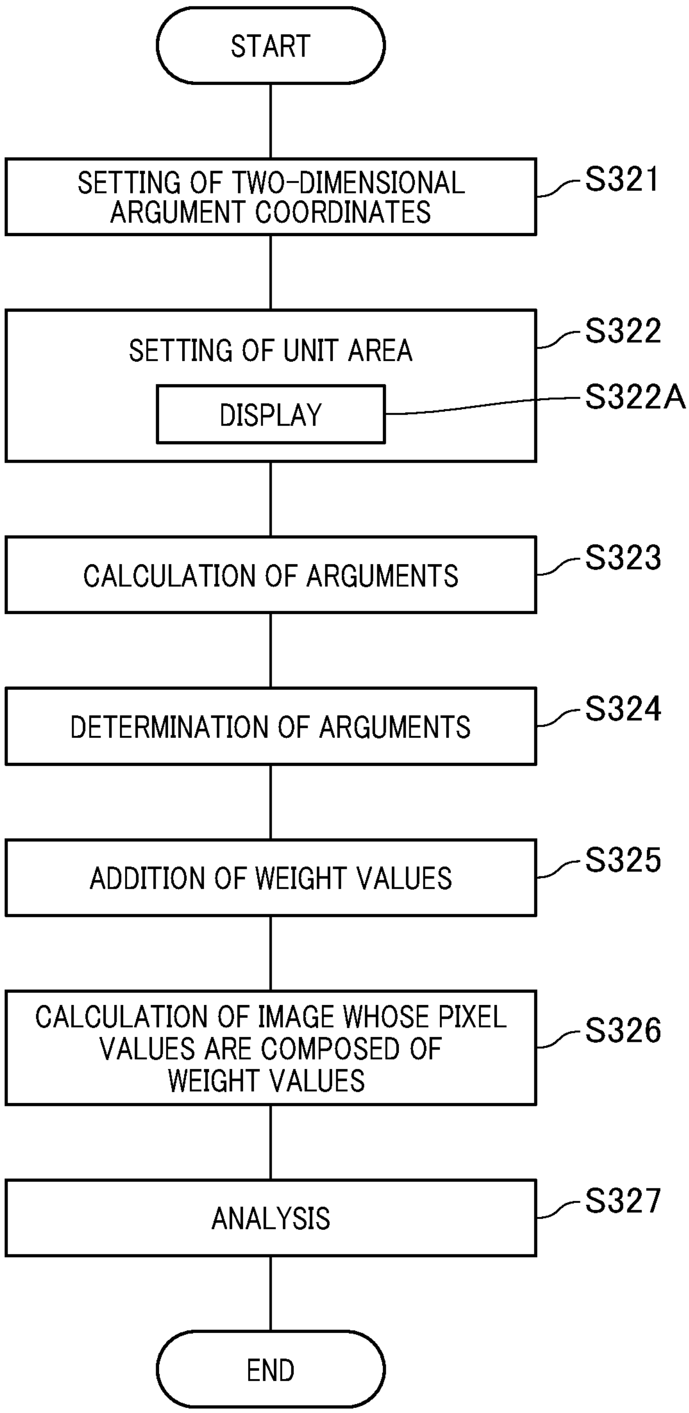
START
SETTING OF TWO-DIMENSIONAL ARGUMENT COORDINATES
S321
SETTING OF UNIT AREA
S322
DISPLAY
S322A
CALCULATION OF ARGUMENTS
S323
DETERMINATION OF ARGUMENTS
S324
ADDITION OF WEIGHT VALUES
S325
CALCULATION OF IMAGE WHOSE PIXEL VALUES ARE COMPOSED OF WEIGHT VALUES
S326
ANALYSIS
S327
END

FIG.9

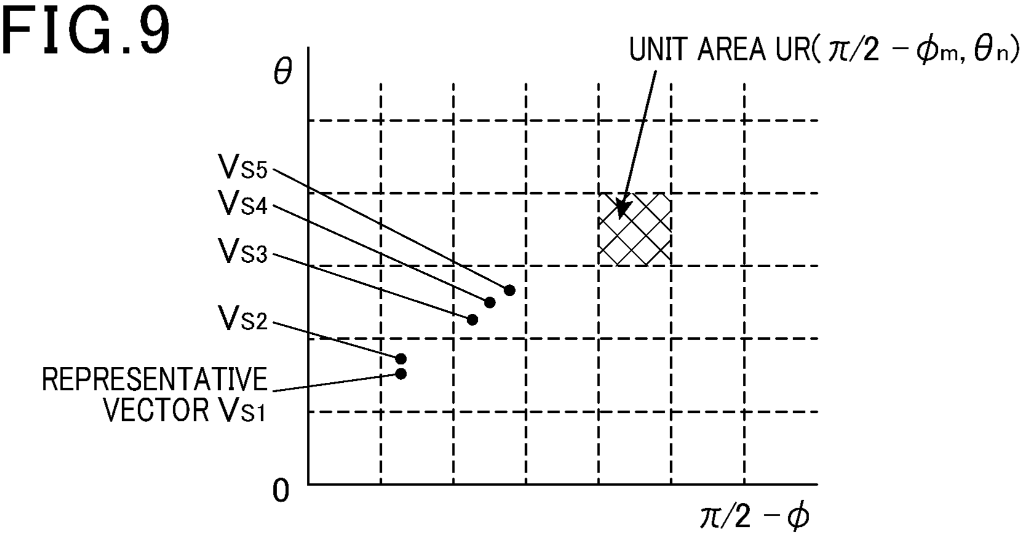


FIG.10

(A)

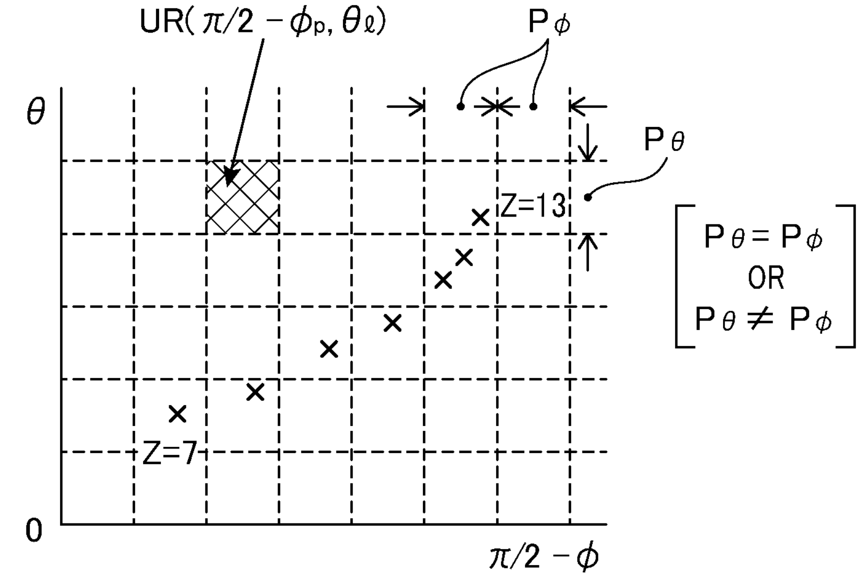


(B)

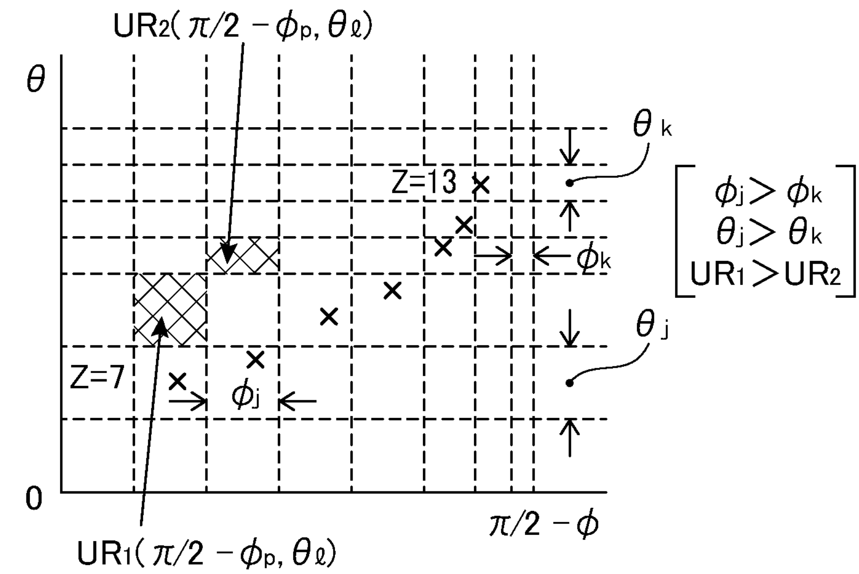

FIG.11

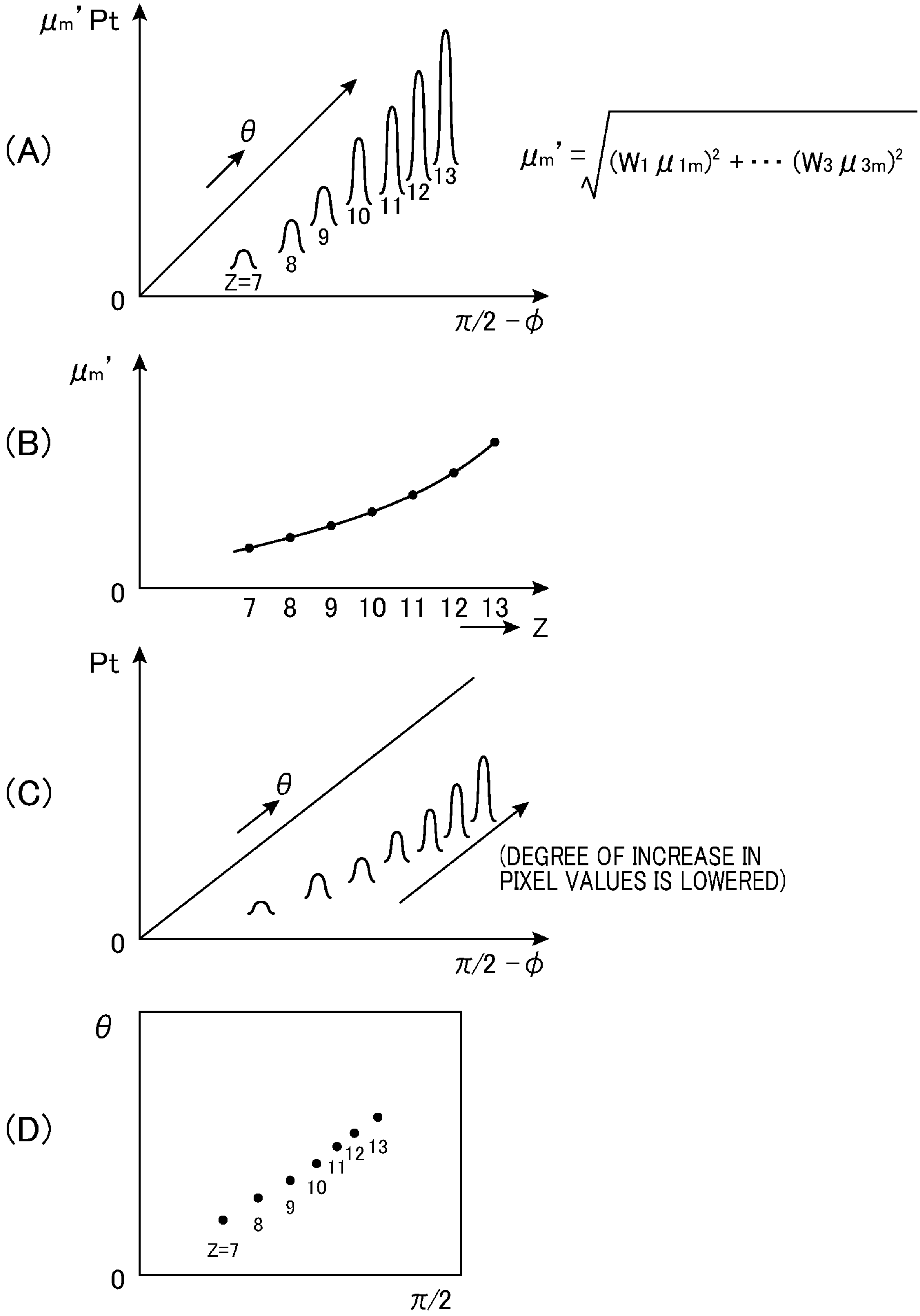
(A)
μm' Pt
θ
Z=7
8
9
10
11
12
13
0
π/2 −φ
μm' = √((W1 μ1m)² + ・・・ (W3 μ3m)²)
(B)
μm'
0
7 8 9 10 11 12 13
Z
(C)
Pt
θ
(DEGREE OF INCREASE IN PIXEL VALUES IS LOWERED)
0
π/2 −φ
(D)
θ
Z=7
8
9
10
11
12
13
0
π/2

FIG.12

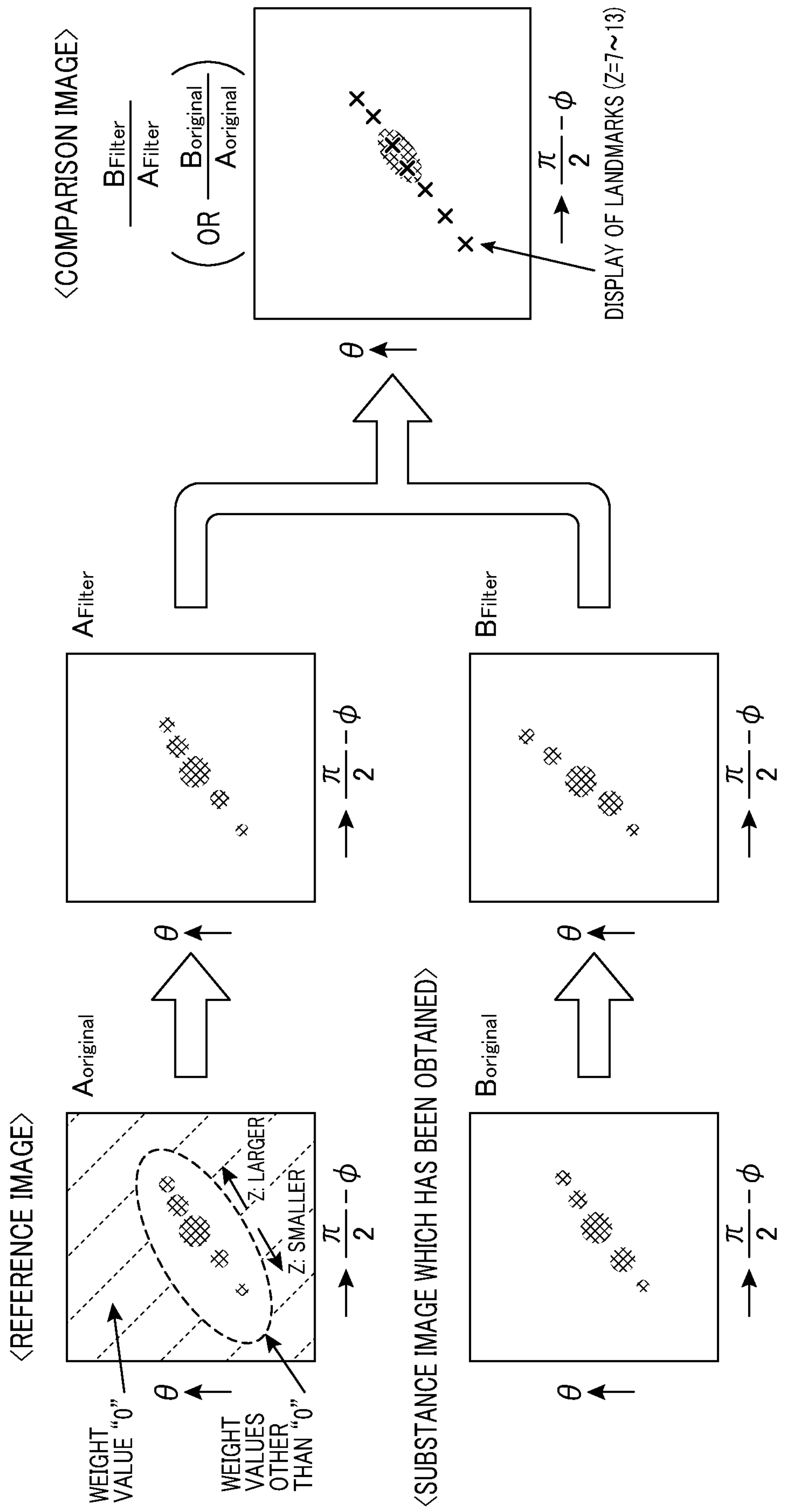

〈REFERENCE IMAGE〉
Aoriginal
WEIGHT VALUE "0"
WEIGHT VALUES OTHER THAN "0"
Z: LARGER
Z: SMALLER
θ
π/2 - φ
〈SUBSTANCE IMAGE WHICH HAS BEEN OBTAINED〉
Boriginal
AFilter
BFilter
〈COMPARISON IMAGE〉
BFilter / AFilter
(OR Boriginal / Aoriginal)
DISPLAY OF LANDMARKS (Z=7~13)

FIG.13

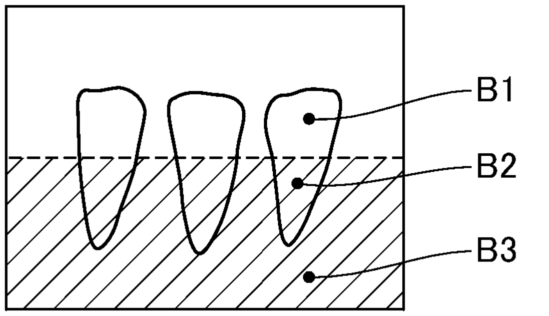
B1
B2
B3

FIG.14

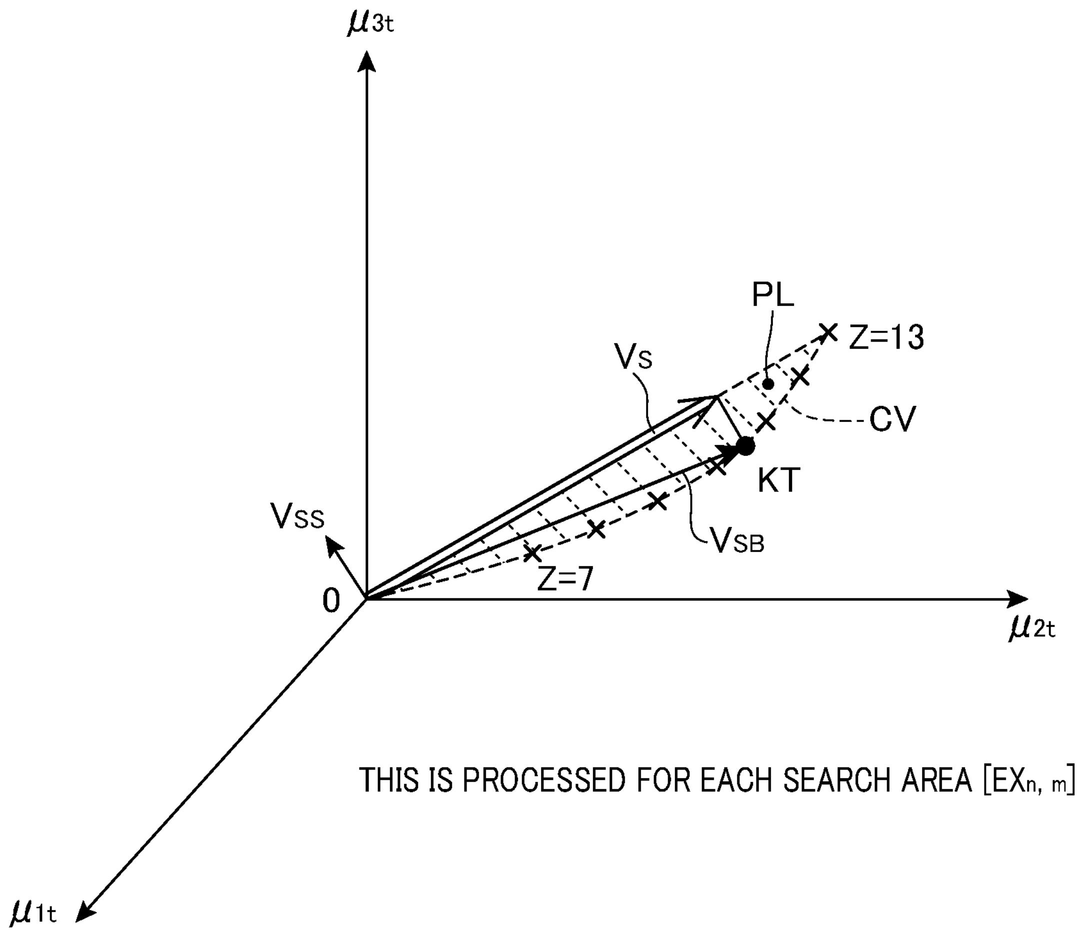
μ3t
PL
Z=13
Vs
CV
KT
Vss
VSB
0
Z=7
μ2t
THIS IS PROCESSED FOR EACH SEARCH AREA [EXn, m]
μ1t

## INTERNATIONAL SEARCH REPORT

International application No.
PCT/JP2020/020870

A. CLASSIFICATION OF SUBJECT MATTER
Int.Cl. A61B6/00(2006.01)i, A61B6/03(2006.01)i, A61B6/14(2006.01)i, G01N23/044(2018.01)i, G01N23/087(2006.01)i
FI: A61B6/00333, A61B6/14300, G01N23/087, G01N23/044, A61B6/03373
According to International Patent Classification (IPC) or to both national classification and IPC

B. FIELDS SEARCHED
Minimum documentation searched (classification system followed by classification symbols)
Int.Cl. A61B6/00-6/14, G01N23/044, G01N23/087

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | WO 2018/212217 A1 (JOB CORPORATION) 22.11.2018 (2018-11-22), entire text, all drawings | 1-37 |
| A | WO 2018/235823 A1 (JOB CORPORATION) 27.12.2018 (2018-12-27), entire text, all drawings | 1-37 |

☐ Further documents are listed in the continuation of Box C.
☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 09.07.2020 | 21.07.2020 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**
Information on patent family members

International application No.
PCT/JP2020/020870

| | | |
|---|---|---|
| WO 2018/212217 A1 | 22.11.2018 | US 2019/0162679 A1 |
| WO 2018/235823 A1 | 27.12.2018 | KR 10-2020-0002918 A |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2013119000 A
- WO 2016171186 A1
- WO 2015111728 A1


### Non-patent literature cited in the description


- Development of Dual-Energy X-Ray Inspection System. *Anritsu Technical,* March 2012, (87